# EUROPEAN PATENT APPLICATION

(11) **EP 3 904 330 A1**
(43) Date of publication of application: **03.11.2021**
(21) Application number: 19903000.8
(22) Date of filing: 26.12.2019
(51) Int. Cl.: C07C 211/01, C07C 211/33, C07C 211/00, A61P 31/06

(54) **ETHYLENEDIAMINE COMPOUND AND USE THEREOF**

(30) Priority: 26.12.2018 CN 201811599918
(71) Applicant: Academy Of Military Medical Sciences, Haidian District Beijing 100850 (CN)
(72) Inventor: ZHONG, Wu, Beijing 100850 (CN); LI, Song, Beijing 100850 (CN); XIAO, Hongjie, Beijing 100850 (CN); ZHOU, Mengdie, Beijing 100850 (CN)
(74) Representative: Inspicos P/S
(86) International application number: PCT/CN2019/128639
(87) International publication number: WO 2020/135569

(57) **Abstract**

The present invention relates to the field of medicinal chemistry, and relates to an ethylenediamine compound represented by Formula A, pharmaceutically acceptable salts, stereoisomers, tautomers or isomer mixtures, hydrates thereof, solvates thereof, or prodrugs thereof, and use thereof in the treatment of tuberculosis.

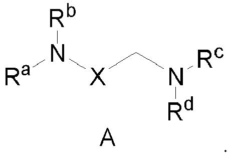

## Description

The present application is based on and claims the benefit of priority from Chinese application No. 201811599918.7, filed on December 26, 2018, the disclosures of which are incorporated herein by reference in its entirety.

### Technical Field

The present invention belongs to the field of medicinal chemistry and relates to an ethylenediamine compound, a pharmaceutically acceptable salt, a stereoisomer, a tautomer or an isomer mixture, a hydrate, a solvate or a prodrug thereof, and use thereof for the treatment of tuberculosis.

### Background Art

Tuberculosis (TB) is a chronic infectious disease caused by *Mycobacterium tuberculosis.* According to estimates of the World Health Organization (WHO), about 2 million people die of tuberculosis every year in the world. Although the existing anti-tuberculosis drugs have largely curbed the spread of tuberculosis, their treatment cycles are too long and patient compliance is poor, and with the increase in the incidence of multi drug-resistant tuberculosis and Extensive Drug Resistant tuberculosis, as well as TB complicated with HIV/AIDS, the TB epidemic is on the rise again, making TB a major public health and social issue of global concern.

Early animal experiments and clinical studies have shown that SQ109 has good effects on drug-sensitive *Mycobacterium tuberculosis,* multidrug-resistant *Mycobacterium tuberculosis* and Extensive Drug Resistant *Mycobacterium tuberculosis,* and it was approved by the FDA and the European Medicines Agency as an "orphan drug" for the treatment of drug-resistant tuberculosis in 2007. Mechanism studies have shown that SQ109 acts on the MmpL3 transmembrane protein of *Mycobacterium tuberculosis,* so as to inhibit the generation of TDM, cause mycolate to fail to link arabinogalactan on the cell wall, thereby inhibiting the synthesis of the cell wall of *Mycobacterium tuberculosis.*

### Contents of the Invention

The present application has designed and synthesized a series of ethylenediamine compounds for the treatment of tuberculosis on the basis of SQ109, and evaluated its anti-tuberculosis activity *in vitro,* showing good in vitro inhibitory activity. The purpose of the present application is to provide an ethylenediamine compound, its pharmaceutically acceptable salt, and its use in the treatment of drug-resistant *Mycobacterium tuberculosis.*

The first aspect of the present application provides a compound represented by Formula A, a pharmaceutically acceptable salt, a stereoisomer, a tautomer or an isomer mixture, a hydrate, a solvate or a prodrug thereof, 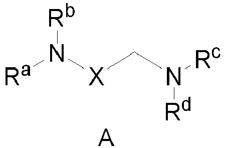
wherein, R^{a} is hydrogen, C₁₋₆ alkyl, C₃₋₁₄ cycloalkyl, 6- to 14-membered aryl, 3- to 14-membered heterocyclyl, 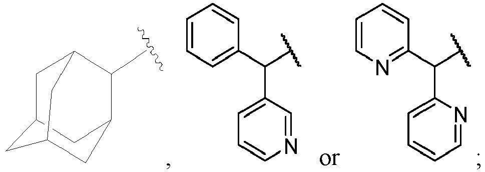
R^{b} is hydrogen, C₁₋₁₀ alkyl, C₃₋₁₄ cycloalkyl, C₂₋₁₂ alkenyl or polyenyl, C₂₋₁₂ alkenylacyl or polyenylacyl, C₂₋₁₀ alkylacyl, substituted or unsubstituted 6- to 14-membered aryl, substituted or unsubstituted 3- to 14-membered heterocyclyl, substituted or unsubstituted 7- to 12-membered bridged-ring group, phenyl-substituted C₁₋₆ alkyl, or indol-3-yl-(CH₂)ₙ-C(O)-, wherein n is an integer between 0 and 4;
X is -CH₂-, -CHR₂- or -C(O)-, wherein R₂ is C₁₋₆ alkyl;
R^{c} is hydrogen, C₁₋₁₀ alkyl, C₃₋₁₄ cycloalkyl, C₂₋₁₀ alkylacyl, C₂₋₁₂ alkenyl or polyenyl, C₂₋₁₂ alkenylacyl or polyenylacyl, substituted or unsubstituted sulfonyl, substituted or unsubstituted 6-to 14-membered aryl, substituted or unsubstituted 3- to 14-membered heterocyclyl, substituted or unsubstituted 7- to 12-membered bridged-ring group;
R^{d} is hydrogen, C₁₋₁₀ alkyl, C₃₋₁₄ cycloalkyl, C₂₋₁₂ alkenyl or polyenyl, C₂₋₁₂ alkenylacyl or polyenylacyl, C₂₋₁₂ alkylacyl, substituted or unsubstituted 6- to 14-membered aryl, substituted or unsubstituted 3- to 14-membered heterocyclyl, substituted or unsubstituted 7- to 12-membered bridged-ring group, phenyl-substituted C₁₋₁₀ alkyl, substituted or unsubstituted sulfonyl, substituted or unsubstituted benzoyl, indol-3-yl-(CH₂)ₙ-C(O)-, wherein n is an integer between 0 and 4.

In some embodiments, the present application provides the compound represented by the Formula A, a pharmaceutically acceptable salt, a stereoisomer, a tautomer or an isomer mixture, a hydrate, a solvate or a prodrug thereof, wherein,

R^{a} is hydrogen,

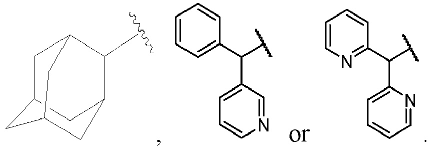

In some embodiments, the present application provides the compound represented by the Formula A, a pharmaceutically acceptable salt, a stereoisomer, a tautomer or an isomer mixture, a hydrate, a solvate or a prodrug thereof, wherein, R^{a} is hydrogen.

In some embodiments, the present application provides the compound represented by the Formula A, a pharmaceutically acceptable salt, a stereoisomer, a tautomer or an isomer mixture, a hydrate, a solvate or a prodrug thereof, wherein,
R^{a} is

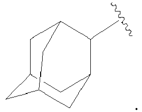

In some embodiments, the present application provides the compound represented by the Formula A, a pharmaceutically acceptable salt, a stereoisomer, a tautomer or an isomer mixture, a hydrate, a solvate or a prodrug thereof, wherein,
R^{a} is

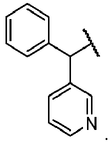

In some embodiments, the present application provides the compound represented by the Formula A, a pharmaceutically acceptable salt, a stereoisomer, a tautomer or an isomer mixture, a hydrate, a solvate or a prodrug thereof, wherein,
R^{a} is

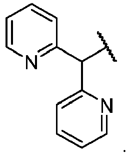

In some embodiments, the present application provides the compound represented by the Formula A, a pharmaceutically acceptable salt, a stereoisomer, a tautomer or an isomer mixture, a hydrate, a solvate or a prodrug thereof, wherein, R^{b} is hydrogen, C₁₋₁₀ alkyl, C₃₋₁₄ cycloalkyl, C₂₋₁₂ alkenyl or polyenyl, C₂₋₁₂ alkenylacyl or polyenylacyl, C₂₋₁₂ alkanylacyl, substituted or unsubstituted 6- to 14-membered aryl, substituted or unsubstituted 3- to 14-membered heterocyclyl, substituted or unsubstituted 7- to 12-membered bridged-ring group.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{b} is hydrogen, C₃₋₁₀ alkyl, C₄₋₁₂ dienyl, phenyl-substituted C₁₋₄ alkyl, or indol-3-yl-(CH₂)ₙ-C(O)-, wherein n is an integer between 0 and 4.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{b} is hydrogen, C₆₋₁₀ alkyl, C₈₋₁₂ dienyl, phenyl-substituted C₁₋₃ alkyl or indol-3-yl-(CH₂)ₙ-C(O)-, wherein n is an integer between 0 and 4.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{b} is hydrogen, n-hexyl, n-heptyl, n-octyl, n-nonyl, n-decyl, 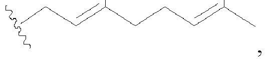 benzyl, phenylethyl, phenyl-n-propyl, phenyl-n-butyl, indol-3-yl-C(O)-, indol-3-yl-CH₂-C(O)-, indol-3-yl-(CH₂)₂-C(O)-, indol-3-yl-(CH₂)₃-C(O)- or indol-3-yl-(CH₂)₄-C(O)-.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{b} is n-hexyl, n-heptyl, n-octyl, n-nonyl or n-decyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{b} is

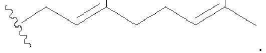

In some embodiments, the present application provides the compound represented by the Formula A, a pharmaceutically acceptable salt, a stereoisomer, a tautomer or an isomer mixture, a hydrate, a solvate or a prodrug thereof, wherein, R^{b} is benzyl or phenylethyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{b} is phenyl-n-propyl or phenyl-n-butyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{b} is indol-3-yl-C(O)-.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{b} is indol-3-yl-CH₂-C(O)-, or indol-3-yl-(CH₂)₂-C(O)-.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{b} is indol-3-yl-(CH₂)₃-C(O)- or indol-3-yl-(CH₂)₄-C(O)-.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{b} is hydrogen, 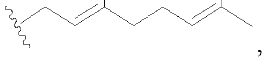 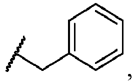 -CH₂(CH₂)₆CH₃, or

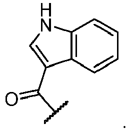

In some embodiments, the present application provides the compound represented by the Formula A, a pharmaceutically acceptable salt, a stereoisomer, a tautomer or an isomer mixture, a hydrate, a solvate or a prodrug thereof, wherein, X is -CH₂- or -C(O)-.

In some embodiments, the present application provides the compound represented by the Formula A, a pharmaceutically acceptable salt, a stereoisomer, a tautomer or an isomer mixture, a hydrate, a solvate or a prodrug thereof, wherein, X is -CH₂-.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{c} is hydrogen, C₄₋₁₂ dienyl, C₄₋₁₂ dienylacyl, C₁₋₄ alkyl-substituted sulfonyl, C₂₋₁₀ alkylacyl, C₃₋₁₀ alkyl, C₃₋₆ cycloalkyl, C₁₋₄ alkyl-phenyl-substituted sulfonyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{c} is hydrogen, C₈₋₁₂ dienyl, C₈₋₁₂ dienylacyl, C₁₋₃ alkyl-substituted sulfonyl, C₁₋₂ alkyl-phenyl-substituted sulfonyl, C₆₋₁₀ alkyl, C₃₋₄ cycloalkyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{c} is hydrogen, methylsulfonyl, ethylsulfonyl, propylsulfonyl, butylsulfonyl, p-toluenesulfonyl, p-ethylbenzenesulfonyl, p-n-propylbenzenesulfonyl, n-hexyl, n-heptyl, n-octyl, n-nonyl, n-decyl, cyclopropyl, cyclobutyl,

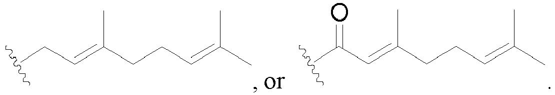

In some embodiments, the present application provides the compound represented by the Formula A, a pharmaceutically acceptable salt, a stereoisomer, a tautomer or an isomer mixture, a hydrate, a solvate or a prodrug thereof, wherein, R^{c} is hydrogen.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{c} is methylsulfonyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{c} is ethyl sulfonyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{c} is propyl sulfonyl or butyl sulfonyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{c} is p-toluenesulfonyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{c} is p-ethylbenzenesulfonyl or p-n-propylbenzenesulfonyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{c} is n-hexyl or n-octyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{c} is n-heptyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{c} is n-nonyl or n-decyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{c} is cyclopropyl, or cyclobutyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{c} is

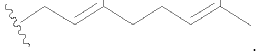

In some embodiments, the present application provides the compound represented by the Formula A, a pharmaceutically acceptable salt, a stereoisomer, a tautomer or an isomer mixture, a hydrate, a solvate or a prodrug thereof, wherein, R^{c} is

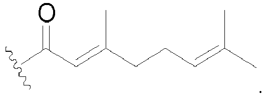

In some embodiments, the present application provides the compound represented by the Formula A, a pharmaceutically acceptable salt, a stereoisomer, a tautomer or an isomer mixture, a hydrate, a solvate or a prodrug thereof, wherein, R^{c} is hydrogen, 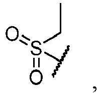 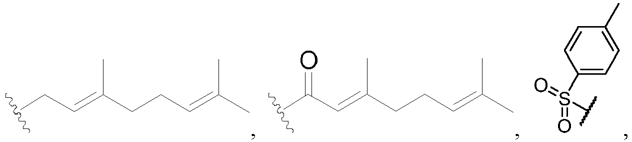 cyclopropyl, -CH₂(CH₂)₆CH₃ or - CH2(CH2)4CH3.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{c} is hydrogen, 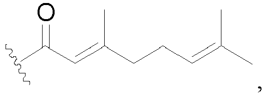 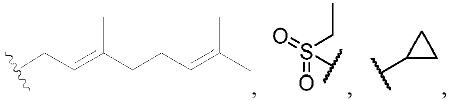 -CH₂(CH₂)₆CH₃, or -CH₂(CH₂)₄CH₃.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{d} is hydrogen, C₁₋₄ alkyl-substituted sulfonyl, C₁₋₄ alkyl-phenyl-substituted sulfonyl, C₄₋₁₂ dienylacyl, benzoyl substituted by one or more methoxy groups, phenyl-substituted C₁₋₄ alkyl, C₃₋₁₀ alkyl, C₂₋₁₂ alkylacyl, C₃₋₆ cycloalkyl, or indol-3-yl-(CH₂)ₙ-C(O)-, wherein n is an integer between 0 and 4.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{d} is hydrogen, C₁₋₃ alkyl-substituted sulfonyl, C₁₋₂ alkyl-phenyl-substituted sulfonyl, C₈₋₁₂ dienylacyl, benzoyl substituted by one or more methoxy groups, phenyl-substituted C₁₋₃ alkyl, C₆₋₁₀ alkyl, C₆₋₁₂ alkylacyl, C₃₋₄ cycloalkyl, or indol-3-yl-(CH₂)ₙ-C(O)-, wherein n is an integer between 0 and 4.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{d} is hydrogen, methylsulfonyl, ethylsulfonyl, propylsulfonyl, butylsulfonyl, p-toluenesulfonyl, p-ethylbenzenesulfonyl, p-n-propylbenzenesulfonyl, 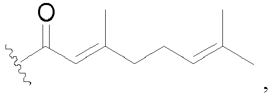 p-methoxybenzoyl, 3,4-dimethoxybenzoyl, 3,4,5-trimethoxybenzoyl, undecanoyl, decanoyl, nonanoyl, octanoyl, heptanoyl, hexanoyl, valeryl, butyryl, benzyl, phenylethyl, phenyl-n-propyl, phenyl-n-butyl, cyclopropyl, cyclobutyl, n-hexyl, n-heptyl, n-octyl, n-nonyl, n-decyl. In some embodiments, the present application provides the compound represented by the Formula A, a pharmaceutically acceptable salt, a stereoisomer, a tautomer or an isomer mixture, a hydrate, a solvate or a prodrug thereof, wherein, R^{d} is hydrogen, 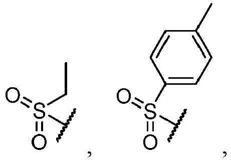 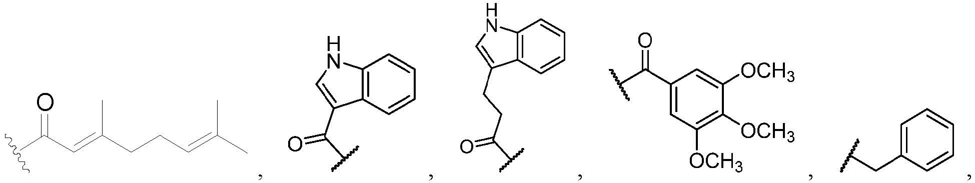 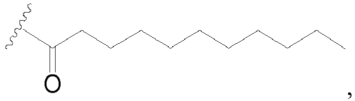 cyclopropyl, -CH₂(CH₂)₆CH₃ or -CH₂(CH₂)₄CH₃.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{d} is hydrogen, 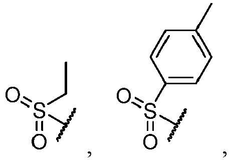 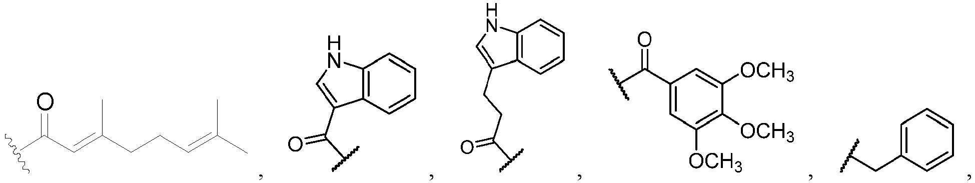 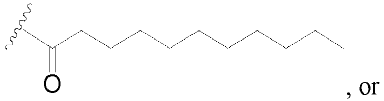 -CH2(CH2)6CH3.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{d} is hydrogen.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{d} is methylsulfonyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{d} is ethyl sulfonyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{d} is propyl sulfonyl or butyl sulfonyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{d} is p-toluenesulfonyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{d} is p-ethylbenzenesulfonyl or p-n-propylbenzenesulfonyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{d} is

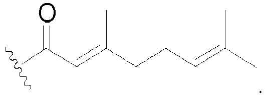

In some embodiments, the present application provides the compound represented by the Formula A, a pharmaceutically acceptable salt, a stereoisomer, a tautomer or an isomer mixture, a hydrate, a solvate or a prodrug thereof, wherein, R^{d} is p-methoxybenzoyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{d} is 3,4-dimethoxybenzoyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{d} is 3,4,5-trimethoxybenzoyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{d} is undecanoyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{d} is decanoyl, nonanoyl or octanoyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{d} is heptanoyl, hexanoyl, valeryl or butyryl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{d} is benzyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{d} is phenylethyl or phenyl-n-propyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{d} is phenyl-n-butyl, cyclopropyl, or cyclobutyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{d} is n-hexyl or n-heptyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{d} is n-octyl or n-nonyl.

In some embodiments, the present application provides the compound represented by the Formula A, a pharmaceutically acceptable salt, a stereoisomer, a tautomer or an isomer mixture, a hydrate, a solvate or a prodrug thereof, wherein, R^{d} is n-decyl.

In some embodiments, the present application provides the compound represented by the Formula A, a pharmaceutically acceptable salt, a stereoisomer, a tautomer or an isomer mixture, a hydrate, a solvate or a prodrug thereof, wherein the compound is represented by Formula I, 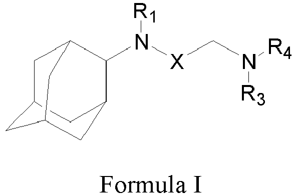 wherein,
R₁ is hydrogen, C₁₋₁₀ alkyl, C₃₋₁₄ cycloalkyl, C₂₋₁₂ alkenyl or polyenyl, C₂₋₁₂ alkenylacyl or polyenylacyl, C₂₋₁₀ alkylacyl, substituted or unsubstituted 6- to 14-membered aryl, substituted or unsubstituted 3- to 14-membered heterocyclyl, substituted or unsubstituted 7- to 12-membered bridged-ring group, phenyl-substituted C₁₋₆ alkyl or indol-3-yl-(CH₂)ₙ-C(O)-, wherein n is an integer between 0 and 4;

   X is -CH₂-, -CHR₂- or -C(O)-, wherein R₂ is C₁₋₆ alkyl;
R₃ is hydrogen, C₁₋₁₀ alkyl, C₃₋₁₄ cycloalkyl, C₂₋₁₂ alkenyl or polyenyl, C₂₋₁₂ alkenylacyl or polyenylacyl, C₂₋₁₀ alkylacyl, substituted or unsubstituted 6- to 14-membered aryl, substituted or unsubstituted 3- to 14-membered heterocyclyl, substituted or unsubstituted 7- to 12-membered bridged-ring group, phenyl-substituted C₁₋₁₀ alkyl, substituted or unsubstituted sulfonyl, substituted or unsubstituted benzoyl, indol-3-yl-(CH₂)ₙ-C(O)-, wherein n is an integer between 0 and 4;
R₄ is hydrogen, C₁₋₁₀ alkyl, C₃₋₁₄ cycloalkyl, C₂₋₁₀ alkylacyl, C₂₋₁₂ alkenyl or polyenyl, C₂₋₁₂ alkenylacyl or polyenylacyl, substituted or unsubstituted sulfonyl, substituted or unsubstituted 6-to 14-membered aryl, substituted or unsubstituted 3- to 14-membered heterocyclyl, substituted or unsubstituted 7- to 12-membered bridged-ring group.

In some embodiments, the present application provides the compound represented by the Formula I, a pharmaceutically acceptable salt, a stereoisomer, a tautomer or an isomer mixture, a hydrate, a solvate or a prodrug thereof, wherein,
R₁ is hydrogen, C₁₋₁₀ alkyl, C₃₋₁₄ cycloalkyl, C₂₋₁₂ alkenyl or polyenyl, C₂₋₁₂ alkenylacyl or polyenylacyl, C₂₋₁₂ alkylacyl, substituted or unsubstituted 6- to 14-membered aryl, substituted or unsubstituted 3- to 14-membered heterocyclyl, substituted or unsubstituted 7- to 12-membered bridged-ring group;
R₃ is hydrogen, C₁₋₁₀ alkyl, C₃₋₁₄ cycloalkyl, C₂₋₁₂ alkenyl or polyenyl, C₂₋₁₂ alkenylacyl or polyenylacyl, C₂₋₁₂ alkylacyl, substituted or unsubstituted 6- to 14-membered aryl, substituted or unsubstituted 3- to 14-membered heterocyclyl, substituted or unsubstituted 7- to 12-membered bridged-ring group.

In some embodiments, the present application provides the compound represented by the Formula I, a pharmaceutically acceptable salt, a stereoisomer, a tautomer or an isomer mixture, a hydrate, a solvate or a prodrug thereof, wherein, X is -CH₂- or -C(O)-.

In some embodiments, the present application provides the compound represented by the Formula I, a pharmaceutically acceptable salt, a stereoisomer, a tautomer or an isomer mixture, a hydrate, a solvate or a prodrug thereof, wherein, R₁ is hydrogen, C₃₋₁₀ alkyl, C₄₋₁₂ dienyl, phenyl-substituted C₁₋₄ alkyl or indol-3-yl-(CH₂)ₙ-C(O)-, wherein n is an integer between 0 and 4. For example, R₁ is hydrogen, C₆₋₁₀ alkyl, C₈₋₁₂ dienyl, phenyl-substituted C₁₋₃ alkyl or indol-3-yl-(CH₂)ₙ-C(O)-, wherein n is an integer between 0 and 4.

In some embodiments, the present application provides the compound represented by the Formula I, a pharmaceutically acceptable salt, a stereoisomer, a tautomer or an isomer mixture, a hydrate, a solvate or a prodrug thereof, wherein R₁ is hydrogen, n-hexyl, n-heptyl, n-octyl, n-nonyl, n-decyl, 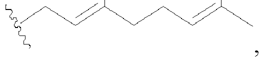 benzyl, phenylethyl, phenyl-n-propyl, phenyl-n-butyl, indol-3-yl-C(O)-, indol-3-yl-CH₂-C(O)-, indol-3-yl-(CH₂)₂-C(O)-, indol-3-yl-(CH₂)₃-C(O)- or indol-3-yl-(CH₂)₄-C(O)-.

In some embodiments, the present application provides the compound represented by the Formula I, a pharmaceutically acceptable salt, a stereoisomer, a tautomer or an isomer mixture, a hydrate, a solvate or a prodrug thereof, wherein R₁ is hydrogen, 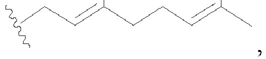 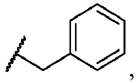 -CH₂(CH₂)₆CH₃ or

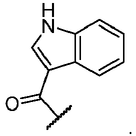

In some embodiments, the present application provides the compound represented by the Formula I, a pharmaceutically acceptable salt, a stereoisomer, a tautomer or an isomer mixture, a hydrate, a solvate or a prodrug thereof, wherein R₁ is hydrogen.

In some embodiments, the present application provides the compound represented by the Formula I, a pharmaceutically acceptable salt, a stereoisomer, a tautomer or an isomer mixture, a hydrate, a solvate or a prodrug thereof, wherein R₁ is n-hexyl or n-heptyl.

In some embodiments, the present application provides the compound represented by the Formula I, a pharmaceutically acceptable salt, a stereoisomer, a tautomer or an isomer mixture, a hydrate, a solvate or a prodrug thereof, wherein R₁ is n-octyl.

In some embodiments, the present application provides the compound represented by the Formula I, a pharmaceutically acceptable salt, a stereoisomer, a tautomer or an isomer mixture, a hydrate, a solvate or a prodrug thereof, wherein R₁ is n-nonyl or n-decyl.

In some embodiments, the present application provides the compound represented by the Formula I, a pharmaceutically acceptable salt, a stereoisomer, a tautomer or an isomer mixture, a hydrate, a solvate or a prodrug thereof, wherein R₁ is

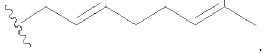

In some embodiments, the present application provides the compound represented by the Formula I, a pharmaceutically acceptable salt, a stereoisomer, a tautomer or an isomer mixture, a hydrate, a solvate or a prodrug thereof, wherein R₁ is benzyl.

In some embodiments, the present application provides the compound represented by the Formula I, a pharmaceutically acceptable salt, a stereoisomer, a tautomer or an isomer mixture, a hydrate, a solvate or a prodrug thereof, wherein R₁ is phenylethyl.

In some embodiments, the present application provides the compound represented by the Formula I, a pharmaceutically acceptable salt, a stereoisomer, a tautomer or an isomer mixture, a hydrate, a solvate or a prodrug thereof, wherein R₁ is phenyl-n-propyl or phenyl-n-butyl.

In some embodiments, the present application provides the compound represented by the Formula I, a pharmaceutically acceptable salt, a stereoisomer, a tautomer or an isomer mixture, a hydrate, a solvate or a prodrug thereof, wherein R₁ is indol-3-yl-C(O)-.

In some embodiments, the present application provides the compound represented by the Formula I, a pharmaceutically acceptable salt, a stereoisomer, a tautomer or an isomer mixture, a hydrate, a solvate or a prodrug thereof, wherein R₁ is indol-3-yl-CH₂-C(O)-.

In some embodiments, the present application provides the compound represented by the Formula I, a pharmaceutically acceptable salt, a stereoisomer, a tautomer or an isomer mixture, a hydrate, a solvate or a prodrug thereof, wherein R₁ is indol-3-yl-(CH₂)₂-C(O)-.

In some embodiments, the present application provides the compound represented by the Formula I, a pharmaceutically acceptable salt, a stereoisomer, a tautomer or an isomer mixture, a hydrate, a solvate or a prodrug thereof, wherein R₁ is indol-3-yl-(CH₂)₃-C(O)- or indol-3-yl-(CH₂)₄-C(O)-.

In some embodiments, the present application provides the compound represented by the Formula I, a pharmaceutically acceptable salt, a stereoisomer, a tautomer or an isomer mixture, a hydrate, a solvate or a prodrug thereof, wherein, R₃ is hydrogen, C₁₋₄ alkyl-substituted sulfonyl, C₁₋₄ alkyl-phenyl-substituted sulfonyl, C₄₋₁₂ dienylacyl, benzoyl substituted by one or more methoxy groups, phenyl-substituted C₁₋₄ alkyl, C₃₋₁₀ alkyl, C₂₋₁₂ alkylacyl, C₃₋₆ cycloalkyl, or indol-3-yl-(CH₂)ₙ-C(O)-, wherein n is an integer between 0 and 4.

In some embodiments, the present application provides the compound represented by the Formula I, a pharmaceutically acceptable salt, a stereoisomer, a tautomer or an isomer mixture, a hydrate, a solvate or a prodrug thereof, wherein, R₃ is hydrogen, C₁₋₃ alkyl-substituted sulfonyl, C₁₋₂ alkyl-phenyl-substituted sulfonyl, C₈₋₁₂ dienylacyl, benzoyl substituted by one or more methoxy groups, phenyl-substituted C₁₋₃ alkyl, C₆₋₁₀ alkyl, C₆₋₁₂ alkylacyl, C₃₋₄ cycloalkyl, or indol-3-yl(CH₂)ₙ-C(O)-, wherein n is an integer between 0 and 4.

In some embodiments, the present application provides the compound represented by the Formula I, a pharmaceutically acceptable salt, a stereoisomer, a tautomer or an isomer mixture, a hydrate, a solvate or a prodrug thereof, wherein, R₃ is hydrogen, methylsulfonyl, ethylsulfonyl, propylsulfonyl, butylsulfonyl, p-toluenesulfonyl, p-ethylbenzenesulfonyl, p-n-propylbenzenesulfonyl, 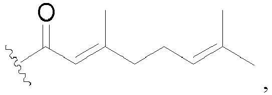 p-methoxybenzoyl, 3,4-dimethoxybenzoyl, 3,4,5-trimethoxybenzoyl, undecanoyl, decanoyl, nonanoyl, octanoyl, heptanoyl, hexanoyl, valeryl, butyryl, benzyl, phenylethyl, phenyl-n-propyl, phenyl-n-butyl, cyclopropyl, cyclobutyl, n-hexyl, n-heptyl, n-octyl, n-nonyl, n-decyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₃ is hydrogen, 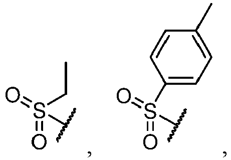 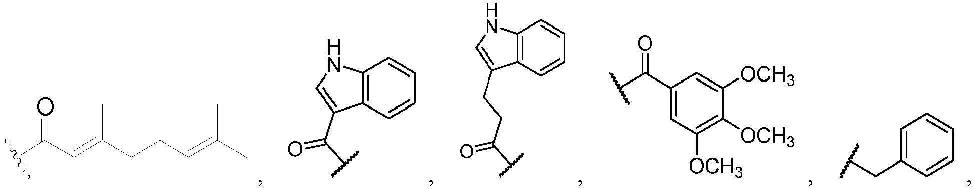 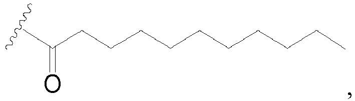 cyclopropyl, -CH₂(CH₂)₆CH₃ or -CH₂(CH₂)₄CH₃.

In some embodiments, the present application provides the compound represented by the Formula I, a pharmaceutically acceptable salt, a stereoisomer, a tautomer or an isomer mixture, a hydrate, a solvate or a prodrug thereof, wherein, R₃ is hydrogen.

In some embodiments, the present application provides the compound represented by the Formula I, a pharmaceutically acceptable salt, a stereoisomer, a tautomer or an isomer mixture, a hydrate, a solvate or a prodrug thereof, wherein, R₃ is methylsulfonyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₃ is ethyl sulfonyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₃ is propyl sulfonyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₃ is butyl sulfonyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₃ is p-toluenesulfonyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₃ is p-ethylbenzenesulfonyl or p-n-propylbenzenesulfonyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₃ is

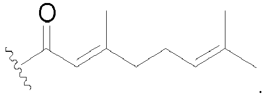

In some embodiments, the present application provides the compound represented by the Formula I, a pharmaceutically acceptable salt, a stereoisomer, a tautomer or an isomer mixture, a hydrate, a solvate or a prodrug thereof, wherein, R₃ is p-methoxybenzoyl or 3,4-dimethoxybenzoyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₃ is 3,4,5-trimethoxybenzoyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₃ is undecanoyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₃ is decanoyl, nonanoyl or octanoyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₃ is heptanoyl or hexanoyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₃ is valeryl or butyryl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₃ is benzyl, phenylethyl, phenyl-n-propyl, phenyl-n-butyl, cyclopropyl, cyclobutyl, n-hexyl, n-heptyl, n-octyl, n-nonyl, or n-decyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₄ is hydrogen, C₄₋₁₂ dienyl, C₄₋₁₂ dienylacyl, C₁₋₄ alkyl-substituted sulfonyl, C₂₋₁₀ alkylacyl, C₃₋₁₀ alkyl, C₃₋₆ cycloalkyl, or C₁₋₄ alkyl-phenyl-substituted sulfonyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₄ is hydrogen, C₈₋₁₂ dienyl, C₈₋₁₂ dienylacyl, C₁₋₃ alkyl-substituted sulfonyl, C₁₋₂ alkyl-phenyl-substituted sulfonyl, C₆₋₁₀ alkyl, or C₃₋₄ cycloalkyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₄ is hydrogen, methylsulfonyl, ethylsulfonyl, propylsulfonyl, butylsulfonyl, p-toluenesulfonyl, p-ethylbenzenesulfonyl, p-n-propylbenzenesulfonyl, n-hexyl, n-heptyl, n-octyl, n-nonyl, n-decyl, cyclopropyl, cyclobutyl,

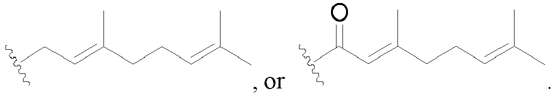

In some embodiments, the present application provides the compound represented by the Formula I, a pharmaceutically acceptable salt, a stereoisomer, a tautomer or an isomer mixture, a hydrate, a solvate or a prodrug thereof, wherein, R₄ is hydrogen, 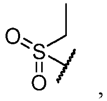 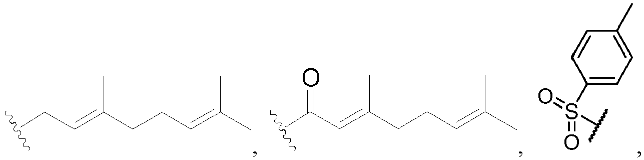 cyclopropyl, -CH₂(CH₂)₆CH₃ or - CH2(CH2)4CH3.

In some embodiments, the present application provides the compound represented by the Formula I, a pharmaceutically acceptable salt, a stereoisomer, a tautomer or an isomer mixture, a hydrate, a solvate or a prodrug thereof, wherein, R₄ is hydrogen.

In some embodiments, the present application provides the compound represented by the Formula I, a pharmaceutically acceptable salt, a stereoisomer, a tautomer or an isomer mixture, a hydrate, a solvate or a prodrug thereof, wherein, R₄ is methylsulfonyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₄ is ethyl sulfonyl or propyl sulfonyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₄ is butyl sulfonyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₄ is p-toluenesulfonyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₄ is p-ethylbenzenesulfonyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₄ is p-n-propylbenzenesulfonyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₄ is n-hexyl or n-heptyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₄ is n-octyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₄ is n-nonyl or n-decyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₄ is cyclopropyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₄ is cyclobutyl.

In some embodiments, the present application provides the compound represented by the Formula I, a pharmaceutically acceptable salt, a stereoisomer, a tautomer or an isomer mixture, a hydrate, a solvate or a prodrug thereof, wherein, R₄ is

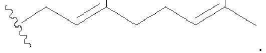

In some embodiments, the present application provides the compound represented by the Formula I, a pharmaceutically acceptable salt, a stereoisomer, a tautomer or an isomer mixture, a hydrate, a solvate or a prodrug thereof, wherein, R₄ is

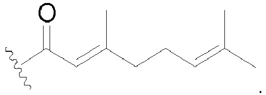

In some embodiments, the present application provides the compound represented by the Formula I, a pharmaceutically acceptable salt, a stereoisomer, a tautomer or an isomer mixture, a hydrate, a solvate or a prodrug thereof, wherein,
R₁ is hydrogen, -CH₂(CH₂)₆CH₃, 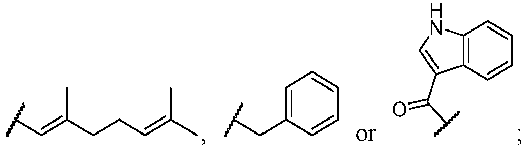
X is -CH₂- or -C(O)-;
R₄ is hydrogen, or 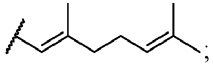
R₃ is hydrogen, -CH₂(CH₂)₄CH₃, -CH₂(CH₂)₆CH₃,

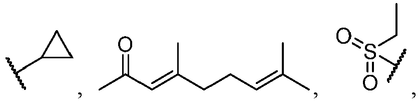

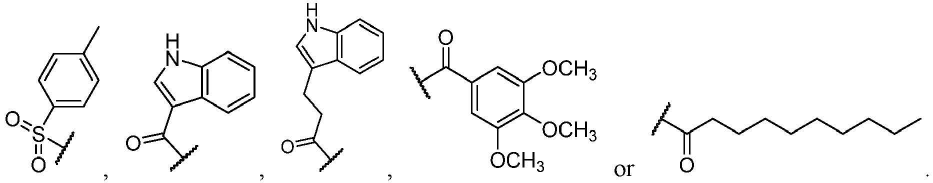

In some embodiments, the present application provides the compound represented by the Formula A, a pharmaceutically acceptable salt, a stereoisomer, a tautomer or an isomer mixture, a hydrate, a solvate or a prodrug thereof, wherein the compound is represented by Formula II, 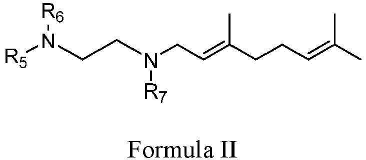 wherein,
R₅ is hydrogen, C₁₋₆ alkyl, C₃₋₁₄ cycloalkyl, 6- to 14-membered aryl, 3- to 14-membered heterocyclyl;
R₆ is hydrogen, C₁₋₆ alkyl, C₃₋₁₄ cycloalkyl, 6- to 14-membered aryl, 3- to 14-membered heterocyclyl, 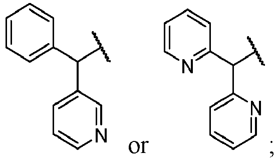
R₇ is hydrogen or substituted or unsubstituted sulfonyl.

In some embodiments, the present application provides the compound represented by the Formula II, a pharmaceutically acceptable salt, a stereoisomer, a tautomer or an isomer mixture, a hydrate, a solvate or a prodrug thereof, wherein, R₅ is hydrogen or C₁₋₄ alkyl.

In some embodiments, the present application provides the compound represented by the Formula II, a pharmaceutically acceptable salt, a stereoisomer, a tautomer or an isomer mixture, a hydrate, a solvate or a prodrug thereof, wherein, R₆ is hydrogen, C₁₋₄ alkyl, 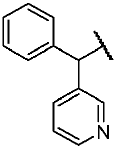 or

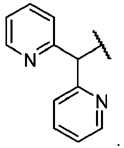

In some embodiments, the present application provides the compound represented by the Formula II, a pharmaceutically acceptable salt, a stereoisomer, a tautomer or an isomer mixture, a hydrate, a solvate or a prodrug thereof, wherein, R₇ is hydrogen, C₁₋₄ alkyl-substituted sulfonyl or C₁₋₄ alkyl-phenyl-substituted sulfonyl.

In some embodiments, the present application provides the compound represented by the Formula II, a pharmaceutically acceptable salt, a stereoisomer, a tautomer or an isomer mixture, a hydrate, a solvate or a prodrug thereof, wherein,
R₅ is hydrogen or C₁₋₄ alkyl;
R₆ is hydrogen, C₁₋₄ alkyl, 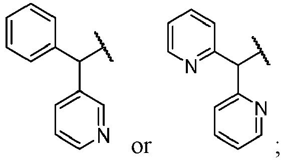
R₇ is hydrogen, C₁₋₄ alkyl-substituted sulfonyl or C₁₋₄ alkyl-phenyl-substituted sulfonyl.

In some embodiments, the present application provides the compound represented by the Formula II, a pharmaceutically acceptable salt, a stereoisomer, a tautomer or an isomer mixture, a hydrate, a solvate or a prodrug thereof, wherein, R₅ is hydrogen, methyl, ethyl, n-propyl or isopropyl.

In some embodiments, the present application provides the compound represented by the Formula II, a pharmaceutically acceptable salt, a stereoisomer, a tautomer or an isomer mixture, a hydrate, a solvate or a prodrug thereof, wherein, R₅ is hydrogen.

In some embodiments, the present application provides the compound represented by the Formula II, a pharmaceutically acceptable salt, a stereoisomer, a tautomer or an isomer mixture, a hydrate, a solvate or a prodrug thereof, wherein, R₅ is methyl or ethyl.

In some embodiments, the present application provides the compound represented by the Formula II, a pharmaceutically acceptable salt, a stereoisomer, a tautomer or an isomer mixture, a hydrate, a solvate or a prodrug thereof, wherein, R₅ is n-propyl or isopropyl.

In some embodiments, the present application provides the compound represented by the Formula II, a pharmaceutically acceptable salt, a stereoisomer, a tautomer or an isomer mixture, a hydrate, a solvate or a prodrug thereof, wherein, R₆ is hydrogen, methyl, ethyl, n-propyl, isopropyl,

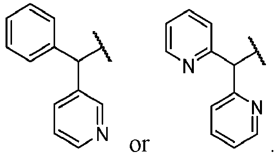

In some embodiments, the present application provides the compound represented by the Formula II, a pharmaceutically acceptable salt, a stereoisomer, a tautomer or an isomer mixture, a hydrate, a solvate or a prodrug thereof, wherein, R₆ is hydrogen.

In some embodiments, the present application provides the compound represented by the Formula II, a pharmaceutically acceptable salt, a stereoisomer, a tautomer or an isomer mixture, a hydrate, a solvate or a prodrug thereof, wherein, R₆ is methyl or ethyl.

In some embodiments, the present application provides the compound represented by the Formula II, a pharmaceutically acceptable salt, a stereoisomer, a tautomer or an isomer mixture, a hydrate, a solvate or a prodrug thereof, wherein, R₆ is n-propyl or isopropyl.

In some embodiments, the present application provides the compound represented by the Formula II, a pharmaceutically acceptable salt, a stereoisomer, a tautomer or an isomer mixture, a hydrate, a solvate or a prodrug thereof, wherein, R₆ is

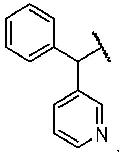

In some embodiments, the present application provides the compound represented by the Formula II, a pharmaceutically acceptable salt, a stereoisomer, a tautomer or an isomer mixture, a hydrate, a solvate or a prodrug thereof, wherein, R₆ is

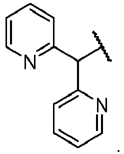

In some embodiments, the present application provides the compound represented by the Formula II, a pharmaceutically acceptable salt, a stereoisomer, a tautomer or an isomer mixture, a hydrate, a solvate or a prodrug thereof, wherein, R₇ is hydrogen, methylsulfonyl, ethylsulfonyl, propylsulfonyl, butylsulfonyl, p-toluenesulfonyl, p-ethylbenzenesulfonyl or p-n-propylbenzenesulfonyl.

In some embodiments, the present application provides the compound represented by the Formula II, a pharmaceutically acceptable salt, a stereoisomer, a tautomer or an isomer mixture, a hydrate, a solvate or a prodrug thereof, wherein, R₇ is hydrogen.

In some embodiments, the present application provides the compound represented by the Formula II, a pharmaceutically acceptable salt, a stereoisomer, a tautomer or an isomer mixture, a hydrate, a solvate or a prodrug thereof, wherein, R₇ is methylsulfonyl.

In some embodiments, the present application provides the compound represented by the Formula II, a pharmaceutically acceptable salt, a stereoisomer, a tautomer or an isomer mixture, a hydrate, a solvate or a prodrug thereof, wherein, R₇ is ethylsulfonyl or propylsulfonyl.

In some embodiments, the present application provides the compound represented by the Formula II, a pharmaceutically acceptable salt, a stereoisomer, a tautomer or an isomer mixture, a hydrate, a solvate or a prodrug thereof, wherein, R₇ is butyl sulfonyl.

In some embodiments, the present application provides the compound represented by the Formula II, a pharmaceutically acceptable salt, a stereoisomer, a tautomer or an isomer mixture, a hydrate, a solvate or a prodrug thereof, wherein, R₇ is p-toluenesulfonyl.

In some embodiments, the present application provides the compound represented by the Formula II, a pharmaceutically acceptable salt, a stereoisomer, a tautomer or an isomer mixture, a hydrate, a solvate or a prodrug thereof, wherein, R₇ is p-ethylbenzenesulfonyl or p-n-propylbenzenesulfonyl.

In some embodiments, the present application provides the compound represented by the Formula II, a pharmaceutically acceptable salt, a stereoisomer, a tautomer or an isomer mixture, a hydrate, a solvate or a prodrug thereof, wherein,
R₅ is hydrogen;
R₆ is hydrogen, 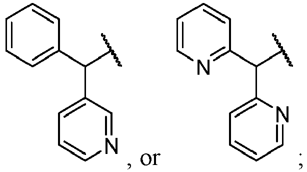
R₇ is hydrogen,

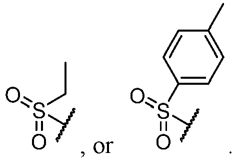

In some embodiments, the present application provides the compound represented by the Formula A, a pharmaceutically acceptable salt, a stereoisomer, a tautomer or an isomer mixture, a hydrate, a solvate or a prodrug thereof, wherein the compound is selected from the group consisting of:

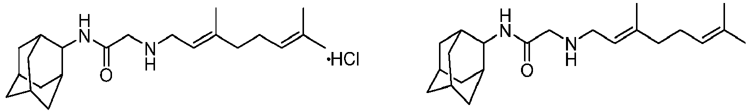

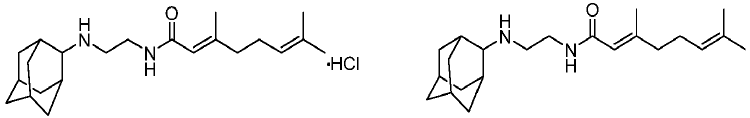

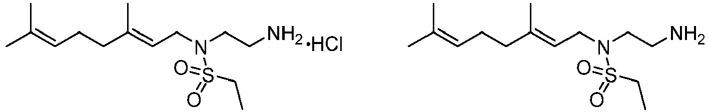

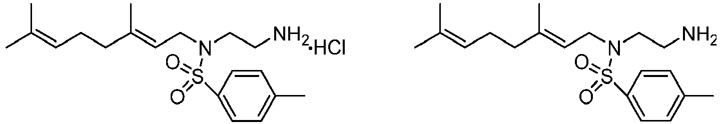

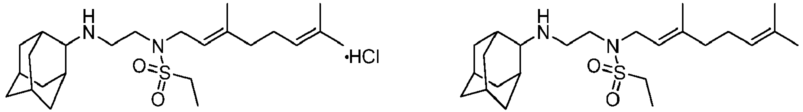

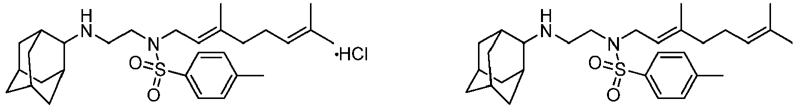

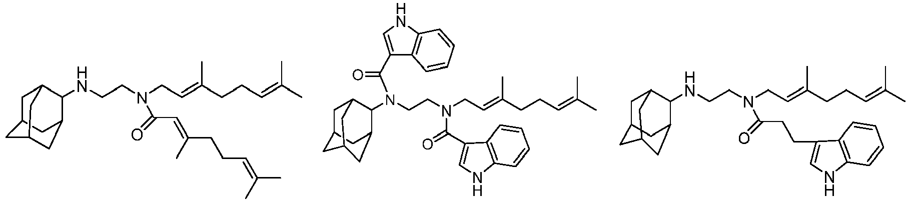

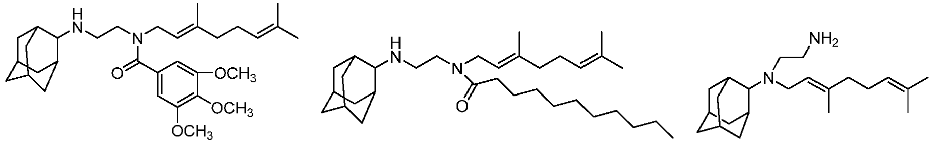

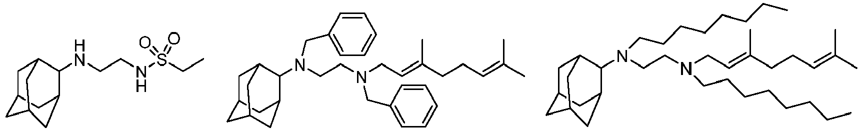

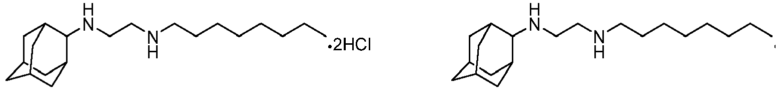

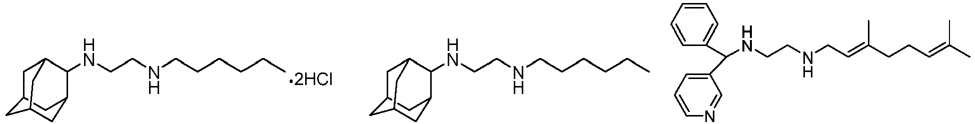

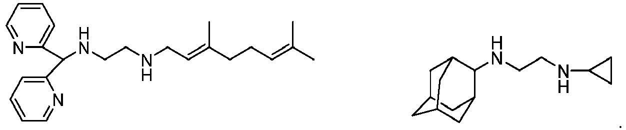

In some embodiments, the present application provides the compound represented by the Formula A, a pharmaceutically acceptable salt, a stereoisomer, a tautomer or an isomer mixture, a hydrate, a solvate or a prodrug thereof, wherein the compound is represented by Formula I', 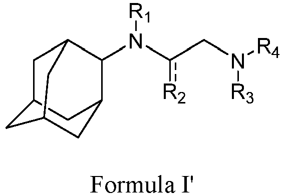 wherein,
R₁ is hydrogen, or is C₁₋₁₀ alkyl, C₃₋₁₄ cycloalkyl, C₂₋₁₂ alkenyl or polyenyl, C₂₋₁₂ alkenylacyl or polyenylacyl, C₂₋₁₀ alkylacyl, substituted or unsubstituted 6- to 14-membered aryl, substituted or unsubstituted 3- to 14-membered heterocyclyl, substituted or unsubstituted 7- to 12-membered bridged-ring group;
R₂ is hydrogen or oxygen, or is C₁₋₆ alkyl;
R₃ is hydrogen, or is C₁₋₁₀ alkyl, C₃₋₁₄ cycloalkyl, C₂₋₁₂ alkenyl or polyenyl, C₂₋₁₂ alkenylacyl or polyenylacyl, C₂₋₁₀ alkylacyl, substituted or unsubstituted 6- to 14-membered aryl, substituted or unsubstituted 3- to 14-membered heterocyclyl, substituted or unsubstituted 7- to 12-membered bridged-ring group;
R₄ is hydrogen, or is C₁₋₁₀ alkyl, C₃₋₁₄ cycloalkyl, C₂₋₁₀ alkylacyl, C₂₋₁₂ alkenyl or polyenyl, C₂₋₁₂ alkenylacyl or polyenylacyl, substituted or unsubstituted sulfonyl, substituted or unsubstituted 6- to 14-membered aryl, substituted or unsubstituted 3- to 14-membered heterocyclyl, substituted or unsubstituted 7- to 12-membered bridged-ring group.

In some embodiments, the present application provides the compound represented by the Formula A, a pharmaceutically acceptable salt, a stereoisomer, a tautomer or an isomer mixture, a hydrate, a solvate or a prodrug thereof, wherein the compound is represented by Formula II', 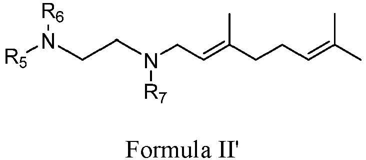 wherein,
R₅ is hydrogen, or is C₁₋₆ alkyl, C₃₋₁₄ cycloalkyl, 6- to 14-membered aryl, or 3- to 14-membered heterocyclyl;
R₆ is hydrogen, or is C₁₋₆ alkyl, C₃₋₁₄ cycloalkyl, 6- to 14-membered aryl, 3- to 14-membered heterocyclyl, 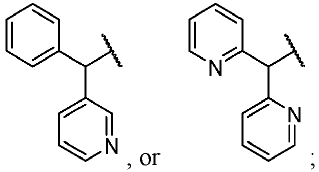
R₇ is hydrogen, or substituted or unsubstituted sulfonyl.

In some embodiments, in the Formula I' described in the present application,
R₁ is hydrogen, -CH₂(CH₂)₆CH₃, 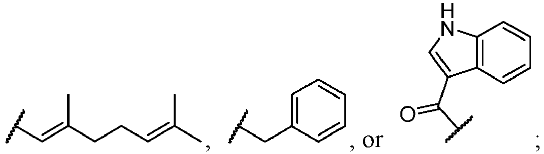
R₂ is hydrogen or oxygen;
R₃ is hydrogen, or 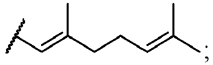
R₄ is hydrogen, -CH₂(CH₂)₄CH₃, -CH₂(CH₂)₆CH₃,

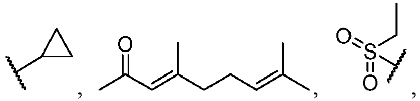

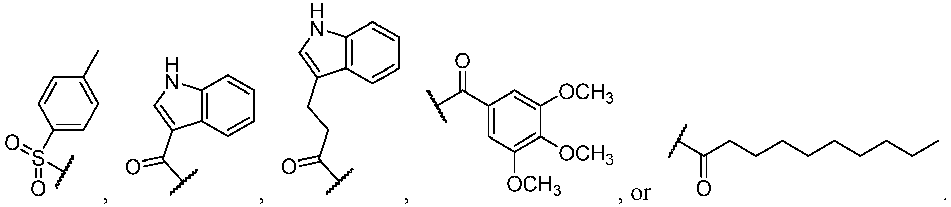

In some embodiments, in the Formula II' described in the present application,
R₅ is hydrogen;
R₆ is hydrogen, 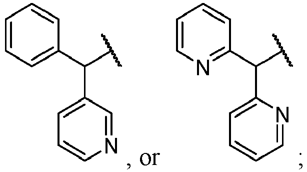
R₇ is hydrogen,

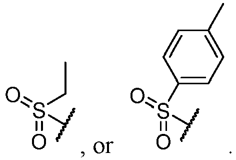

In some embodiments, the present application also provides a pharmaceutically acceptable salt of the ethylenediamine compound represented by Formula A, Formula I, Formula II, Formula I' or Formula II', and the salt is preferably one of conventional inorganic acid salts of the ethylenediamine compound, such as hydrochloride, sulfate, phosphate, as well as organic acid salts, such as methanesulfonate, trifluoromethanesulfonate, acetate, trifluoroacetate, benzoate, preferably hydrochloride.

The ethylenediamine compound represented by Formula A, Formula I, Formula II, Formula I' or Formula II', a pharmaceutically acceptable salt, a stereoisomer, a tautomer or an isomer mixture, a hydrate, a solvate or a prodrug thereof as described in the present application has potential inhibitory activity against drug-resistant *Mycobacterium tuberculosis,* so that such compound can be used as an active ingredient of medicament for the treatment of a disease caused by a drug-resistant *Mycobacterium tuberculosis.*

A use of the ethylenediamine compound represented by the Formula A, Formula I, Formula II, Formula I' or Formula II', a pharmaceutically acceptable salt, a stereoisomer, a tautomer or an isomer mixture, a hydrate, a solvate or a prodrug thereof in the manufacture of a medicament for the treatment of a disease caused by a drug-resistant *Mycobacterium tuberculosis* also falls into the protection scope of the present application.

The second aspect of the present application further provides a pharmaceutical composition, which comprises the compound represented by Formula A, Formula I, Formula II, Formula I' or Formula II', a pharmaceutically acceptable salt, a stereoisomer, a tautomer or an isomer mixture, a hydrate, a solvate or a prodrug thereof as described in the present application, and optionally one or more pharmaceutically acceptable carriers or excipients.

The third aspect of the present application provides a use of the compound represented by Formula I or Formula II, a pharmaceutically acceptable salt, a stereoisomer, a tautomer or an isomer mixture, a hydrate, a solvate or a prodrug thereof as described in the present application in inhibition of *Mycobacterium tuberculosis* (e.g., drug-resistant *Mycobacterium tuberculosis*)*.*

The fourth aspect of the present application provides a use of the compound represented by Formula A, Formula I, Formula II, Formula I' or Formula II', a pharmaceutically acceptable salt, a stereoisomer, a tautomer or an isomer mixture, a hydrate, a solvate or a prodrug thereof as described in the present application in the manufacture of a medicament for inhibiting the growth or reproduction of *Mycobacterium tuberculosis* (e.g., drug-resistant *Mycobacterium tuberculosis*)*.*

The fifth aspect of the present application provides a use of the compound represented by Formula A, Formula I, Formula II, Formula I' or Formula II', a pharmaceutically acceptable salt, a stereoisomer, a tautomer or an isomer mixture, a hydrate, a solvate or a prodrug thereof as described in the present application in the manufacture of a medicament for treating a tuberculosis.

The sixth aspect of the present application provides the compound represented by Formula A, Formula I, Formula II, Formula I' or Formula II', a pharmaceutically acceptable salt, a stereoisomer, a tautomer or an isomer mixture, a hydrate, a solvate or a prodrug thereof as described in the present application, for use in inhibiting the growth or reproduction of *Mycobacterium tuberculosis* (e.g., drug-resistant *Mycobacterium tuberculosis*) or treating a tuberculosis.

The seventh aspect of the present application provides a method for treating tuberculosis, comprising administering to a subject in need a therapeutically effective amount of at least one of the compound represented by Formula A, Formula I, Formula II, Formula I' or Formula II', a pharmaceutically acceptable salt, a stereoisomer, a tautomer or an isomer mixture, a hydrate, a solvate or a prodrug thereof as described in the present application.

The eighth aspect of the present application provides a method for inhibiting the growth or reproduction of *Mycobacterium tuberculosis* (e.g., drug-resistant *Mycobacterium tuberculosis*) in vivo or in vitro, comprising contacting the *Mycobacterium tuberculosis* (e.g., drug-resistant *Mycobacterium tuberculosis*) to the compound represented by Formula A, Formula I, Formula II, Formula I' or Formula II', a pharmaceutically acceptable salt, a stereoisomer, a tautomer or an isomer mixture, a hydrate, a solvate or a prodrug thereof as described in the present application.

The compound as described in the present application can be prepared by general chemical synthesis methods.

In some embodiments, when R^{a} is 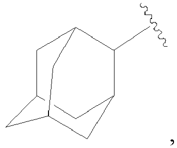 R^{b} is hydrogen, X is -CH₂-, and R^{d} is hydrogen, the compound described in the present application can be prepared by the following method comprising, 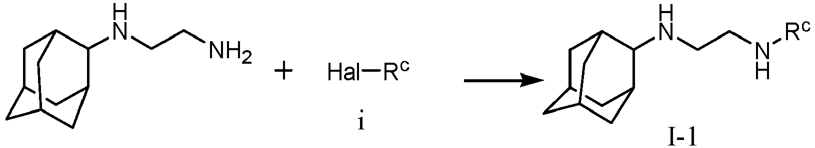 reacting N-adamantan-2-yl-ethane-1,2-diamine with a compound of Formula i to obtain a compound of Formula I-1, wherein Hal is a halogen, such as Cl or Br, and R^{c} is defined as described in the present application.

In some embodiments, when R^{a} is

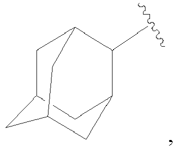

X is -CH₂-, and both R^{c} and R^{d} are hydrogen, the compound described in the present application can be prepared by the following method comprising, 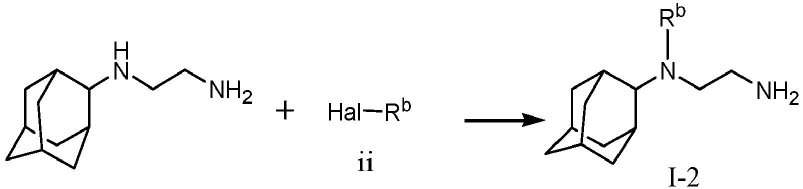 reacting N-adamantan-2-yl-ethane-1,2-diamine with a compound of Formula ii to obtain a compound of Formula 1-2, wherein Hal is a halogen, such as Cl or Br, and R^{b} is defined as described in the present application.

In some embodiments, when both R^{a} and R^{b} are hydrogen, X is -CH₂-, R^{c} is 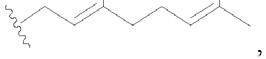 and R^{d} is a substituted or unsubstituted sulfonyl, the compound described in the present application can be prepared by the following method comprising, 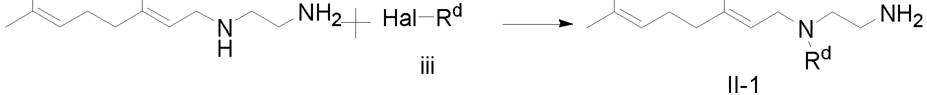 reacting N-geranyl-ethane-1,2-diamine with a compound represented by Formula iii to obtain a compound represented by Formula II-1, wherein Hal is a halogen, such as Cl or Br.

In some embodiments, when R^{a} is 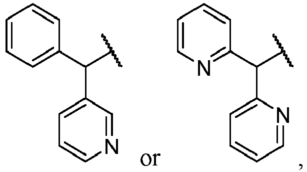 R^{b} is hydrogen, X is -CH₂-, R^{c} is 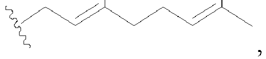 and R^{d} is hydrogen, the compound described in the present application can be prepared by the following method comprising, 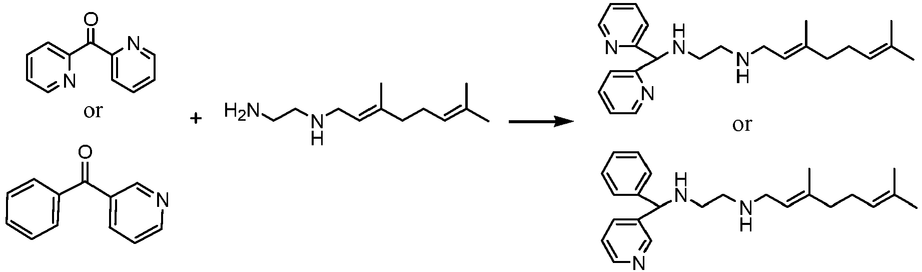 reacting N-geranyl-2-yl-ethane-1,2-diamine with di-2-pyridyl ketone or 3-benzoylpyridine to obtain the target compound.

In some embodiments, when R^{a} is 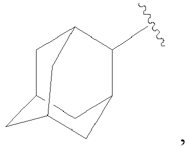 X is -CH₂-, and R^{c} is

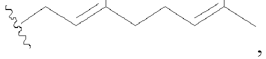

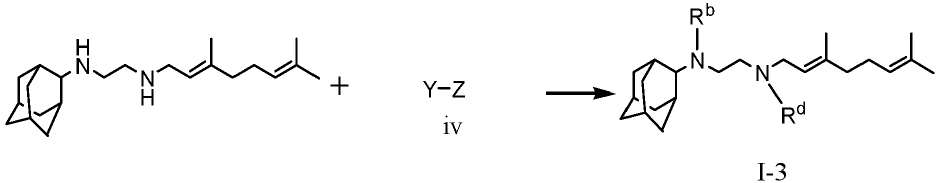

SQ109 is reacted with the compound represented by Formula iv to obtain the target compound, wherein R^{b} is hydrogen, C₁₋₁₀ alkyl, phenyl-substituted C₁₋₆ alkyl or indol-3-yl-(CH₂)ₙ-C(O)-, wherein n is an integer between 0 and 4; R^{d} is C₁₋₁₀ alkyl, C₂₋₁₂ alkenyl or polyenyl, C₂₋₁₂ alkenylacyl or polyenylacyl, C₂₋₁₂ alkylacyl, phenyl-substituted C₁₋₁₀ alkyl, substituted or unsubstituted sulfonyl, substituted or unsubstituted benzoyl, indol-3-yl-(CH₂)ₙ-C(O)-, wherein n is an integer between 0 and 4; Y is halogen (e.g., Cl or Br) or hydroxyl; Z has the same definition as R^{b} or R^{d}.

For specific synthesis examples of the compounds described in the present application, please refer to the examples of the present application.

In some embodiments, the compound represented by Formula A, Formula I, Formula II, Formula I' or Formula II' described in the present application is as follows:

| No. | Structure and chemical name | Molecular formula | Molar mass (g/mol) | Melting point (°C) |
|---|---|---|---|---|
| XHJ-2-37 | 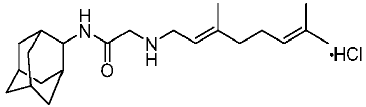 N-adamantan-2-yl-2-(3,7-dimethyl-oct-2,6-dienylamino)-acetamide hydrochloride | C₂₂H₃₇N₂OCl | 380.99 | 168-172 |
| XHJ-2-32 | 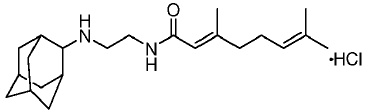 3,7-dimethyl-oct-2,6-dienoic acid [2-(adamantan-2-yl-amino)ethyl]amide hydrochloride | C₂₂H₃₇N₂OCl | 380.99 | 150-164 |
| XHJ-2-39 | 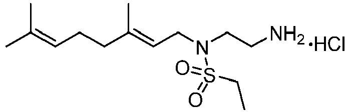 N-geranyl-N-(2-aminoethyl)ethylsulfonamide hydrochloride | C₁₄H₂₉N₂O₂SCl | 324.91 | 103-106 |
| XHJ-2-45 | 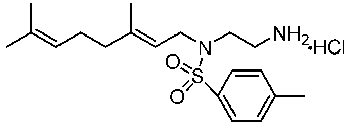 N-geranyl-N-(2-aminoethyl)-p-toluenesulfonamide hydrochloride | C₁₉H₃₁N₂O₂SCl | 386.98 | / |
| XHJ-3-1 | 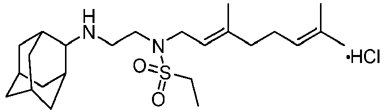 N-adamantan-2-yl-N',N'-ethylsulfonyl-(3,7-dimethyl-oct-2,6-dienyl)-ethane-1,2-diamine hydrochloride | C₂₄H₄₃N₂O₂SCl | 459.13 | 108-112 |
| XHJ-3-3 | 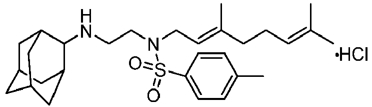 N-adamantan-2-yl-N',N'-p-toluenesulfonyl-(3,7-dimethyl-oct-2,6-dienyl)-ethane-1,2-diamine hydrochloride | C₂₉H₄₅N₂O₂SCl | 521.2 | 179-182 |
| XHJ-3-11 | 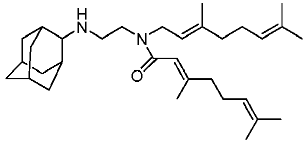 N-adamantan-2-yl-N',N'-(3,7-dimethyl-oct-2,6-dienyl)-(3,7-dimethyl-oct-2,6-dienoyl)-ethane-1,2-diamine | C₃₂H₅₂N₂O | 480.77 | / |
| XHJ-3-15 | 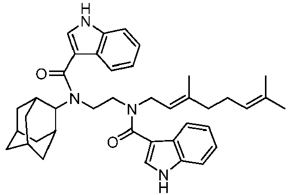 N,N-adamantan-2-yl-(3 -indolylformyl)-N',N'-(3,7-dimethyl-oct-2,6-dienyl)-(3-indolylformyl)-ethane-1,2-diamine | C₄₀H₄₈N₄O₂ | 616.83 | 248-252 |
| XHJ-3-27 | 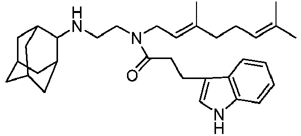 N-adamantan-2-yl-N',N'-(3-indolylpropionyl)-(3,7-dimethyl-oct-2,6-dienyl)-ethane-1,2-diamine | C₃₃H₄₇N₃O | 501.75 | / |
| XHJ-3-29 | 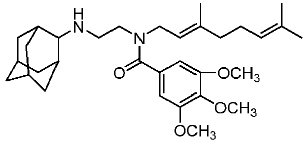 N-adamantan-2-yl-N',N'-(3,4,5-trimethoxybenzoyl)-(3,7-dimethyl-oct-2,6-dienyl)-ethane-1,2-diamine | C₃₂H₄₈N₂O₄ | 524.73 | / |
| XHJ-3-31 | 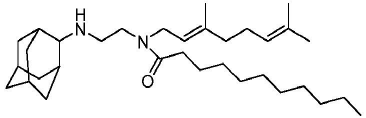 N-adamantan-2-yl-N',N'-undecanoyl-(3,7-dimethyl-oct-2,6-dienyl)-ethane-1,2-diamine | C₃₃H₅₈N₂O | 498.83 | / |
| XHJ-3-45 | 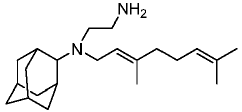 N,N-(adamantan-2-yl)-(3,7-dimethyl-oct-2,6-dienyl)-ethane-1,2-diamine | C22H38N2 | 330.55 | 102-104 |
| XHJ-3-46 | 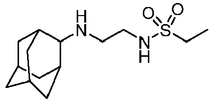 N-(adamantan-2-yl)-N'-ethylsulfonyl-ethane-1,2-diamine | C₁₄H₂₆N₂O₂S | 286.43 | 72-74 |
| XHJ-4-11 | 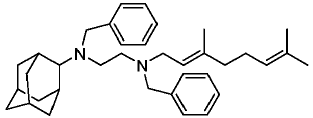 N,N-(adamantan-2-yl)-benzyl-N',N'-(3,7-dimethyl-oct-2,6-dienyl)-benzyl-ethane-1,2-diamine | C₃₆H₅₀N₂ | 510.8 | / |
| XHJ-4-15 | 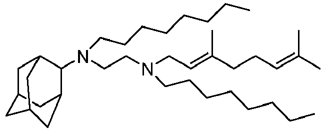 N,N-(adamantan-2-yl)-octyl-N',N'-(3,7-dimethyl-oct-2,6-dienyl)-octyl-ethane-1,2-diamine | C₃₈H₇₀N₂ | 554.98 | / |
| XHJ-5-3 | 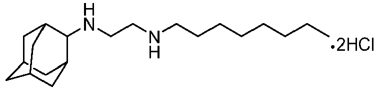 N-(adamantan-2-yl)-N'-octyl-ethane-1,2-diamine dihydrochloride | C₂₀H₃₉Cl₂N₂ | 378.44 | 224-226 |
| XHJ-5-5 | 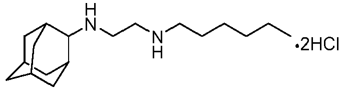 N-(adamantan-2-yl)-N'-hexyl-ethane- 1,2-diamine dihydrochloride | C₁₈H₃₅Cl₂N₂ | 350.39 | 212-216 |
| XHJ-5-8 | 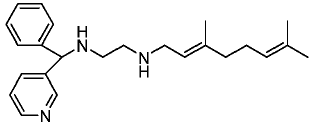 N-(phenyl-pyridin-3-yl-methyl)-N'-(3,7-dimethyl-oct-2,6-dienyl)-ethane-1,2-diamine | C24H33N3 | 363.54 | / |
| XHJ-5-14 | 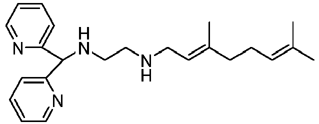 N-(dipyridin-2-yl-methyl)-N'-(3,7-dimethyl-oct-2,6-dienyl)-ethane-1,2-diamine | C23H32N4 | 364.53 | / |
| XHJ-5-18 | 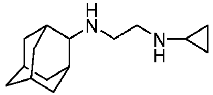 N-(adamantan-2-yl)-N'-cyclopropyl-ethane-1,2-diamine | C₁₅H₂₆N₂ | 234.38 | 197-200 |

| | | | | |
|---|---|---|---|---|
| Note: The sign "/" in the melting point column indicates that the compound is liquid at room temperature, or is obtained by refrigeration and solidification, and its melting point data cannot be obtained. | | | | |

The term "C₁₋₁₀ alkyl" used in the present application refers to a straight or branched chain alkyl having 1 to 10 carbon atoms, such as C₃₋₁₀ alkyl, C₆₋₁₀ alkyl, C₁₋₂ alkyl, C₁ alkyl, C₂ alkyl, C₃ alkyl, C₄ alkyl, C₅ alkyl, C₆ alkyl, C₇ alkyl, C₈ alkyl, C₉ alkyl, C₁₀ alkyl. Specific examples include, but are not limited to, methyl, ethyl, propyl, isopropyl, n-butyl, isobutyl, sec-butyl, tert-butyl, n-pentyl, n-hexyl, n-heptyl, n-octyl, n-nonyl, n-decyl, etc.

The term "C₃₋₁₄ cycloalkyl" used in the present application refers to a saturated cyclic hydrocarbonyl having 3 to 14 carbon atoms and having a monocyclic or bicyclic or multiple condensed rings, for example, having 3 to 4, 5 to 8, 3 to 6 or 5 to 6 carbon atoms. Typical examples of "C₃₋₁₄ cycloalkyl" include, but are not limited to, monocyclic structures such as cyclopropyl, cyclobutyl, cyclopentyl, cyclohexyl, cycloheptyl, cyclooctyl, 1-methylcyclopropyl, 2-methylcyclopentyl, 2-methylcyclooctyl, etc.; bicyclic structures, such as bicyclo[2.2.1]heptyl, etc.

The term "6- to 14-membered aryl" used in the present application refers to an unsaturated aromatic carbocyclic group having 6 to 14 carbon atoms and having one monocyclic ring or two or more condensed rings. The aryl has, for example, 5 to 8 or 5 to 6 carbon atoms. Typical examples of "C₆₋₁₄ aryl" include, but are not limited to, phenyl, naphthyl, anthracenyl and the like.

The term "3- to 14-membered heterocyclyl" used in the present application refers to a saturated or partially saturated and non-aromatic cyclic group containing at least one heteroatom (e.g., containing 1, 2, 3, 4 or 5), having 3 to 14 ring atoms and having monocyclic or bicyclic or multiple condensed rings, wherein the heteroatom is nitrogen atom, oxygen atom and/or sulfur atom. The "3- to 14-membered heterocyclyl" may be oxo- or thio- substituted. For example, the "3- to 14-membered heterocyclyl" described in the present application contains 1 to 2 heteroatoms, for example, contains one nitrogen atom, oxygen atom or sulfur atom, or contains one nitrogen atom and one oxygen atom. The "3- to 14-membered heterocyclyl" includes, for example, "4- to 10-membered heterocyclyl", "4- to 8-membered heterocyclyl", "4- to 6-membered heterocyclyl", "4- to 7-membered heterocyclyl", "5- to 6-membered heterocyclyl", "4- to 6-membered saturated heterocyclyl", "5- to 6-membered saturated heterocyclyl", "4- to 8-membered oxygen-containing heterocyclyl", "4- to 6-membered oxygen-containing heterocyclyl", "5- to 6-membered oxygen-containing heterocyclyl", "4- to 6-membered saturated oxygen-containing heterocyclyl", "4- to 10-membered nitrogen-containing heterocyclyl", "4- to 7-membered nitrogen-containing heterocyclyl", "4- to 8-membered nitrogen-containing heterocyclyl", "5- to 6-membered nitrogen-containing heterocyclyl", "5- to 6-membered saturated nitrogen-containing heterocyclyl" and the like. Specific examples of "3- to 14-membered heterocyclyl" include, but are not limited to: azetidinyl, 1,4-dioxanyl, 1,3-dioxanyl, 1,3-dioxolanyl, 1,4-dioxacyclohexadienyl, tetrahydrofuranyl, dihydropyrrolyl, pyrrolidinyl, imidazolidinyl, 4,5-dihydroimidazolyl, pyrazolidinyl, 4,5-dihydropyrazolyl, 2,5-dihydrothienyl, tetrahydrothienyl, piperazinyl, thiazinyl, piperidinyl, morpholinyl, etc.

The term "7- to 12-membered bridged-ring group" used in the present application refers to a ring system group with 7 to 12 ring atoms formed by two or more cyclic structures sharing two atoms that are not directly connected to each other.

The term "C₂₋₁₂ alkylacyl" used in the present application refers to "alkyl-C(O)-", and the group has 2 to 12 carbon atoms.

The term "alkenyl" as used in the present application refers to a branched and unbranched unsaturated hydrocarbonyl containing at least one double bond.

The term "polyenyl" as used in the present application refers to a branched and unbranched unsaturated hydrocarbonyl containing at least two double bonds.

The term "C₂₋₁₂ alkenyl" used in the present application refers to an alkenyl having 2 to 12 carbon atoms, such as vinyl, 1-methyl-1-vinyl, 2,2-dimethyl-1-vinyl, 1-propenyl, 2-propenyl (allyl), 1-butenyl, 2-butenyl, 3-butenyl, 4-pentenyl, 1-methyl-4-pentenyl, 3-methyl-1-pentenyl, 1-hexenyl, 2-hexenyl and the like.

The term "C₂₋₁₂ polyenyl" used in the present application refers to a polyenyl having 2 to 12 carbon atoms, for example

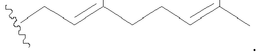

The term "C₂₋₁₂ alkenylacyl" used in the present application refers to "alkenyl-C(O)-", and the group has 2 to 12 carbon atoms.

The term "C₂₋₁₂ polyenylacyl" used in the present application refers to "polyenyl-C(O)-", and the group has 2 to 12 carbon atoms, for example,

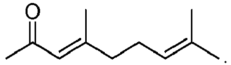

If the chemical name of the compound described in the present application is inconsistent with the structural formula, the compound structural formula shall prevail.

According to the present application, the pharmaceutical composition described in the present application can be administered in any of the following ways: oral, spray inhalation, rectal administration, nasal administration, buccal administration, vaginal administration, topical administration, parenteral administration such as subcutaneous, intravenous, intramuscular, intraperitoneal, intrathecal, intraventricular, intrasternal and intracranial injection or infusion, or administration with the aid of an explanted reservoir.

When orally administered, the compound described in the present application can be prepared into any orally acceptable forms of preparation, including but not limited to, tablets, capsules, aqueous solutions or aqueous suspensions. The carriers for use in a tablet generally include lactose and maize starch, in addition, a lubricant such as magnesium stearate can also be added. Diluents for use in a capsule generally include lactose and dry maize starch. An aqueous suspension is generally obtained by mixing an active ingredient with a suitable emulsifier and a suitable suspending agent. Optionally, a sweetening agent, a flavoring agent or a coloring agent may be added to the above-mentioned forms of oral preparations.

When rectally administrated, the compound described in the present application can generally be prepared into the form of suppositories, which are prepared by mixing the drug with a suitable non-irritating excipient. The excipient presents a solid state at room temperature, but melts at the rectal temperature to release the drug. Such excipient includes cocoa butter, beeswax and polyethylene glycol.

When topically administrated, especially for the treatment of affected surfaces or organs such as eye and skin, or lower intestinal neurological diseases that are easily accessible by topical application, the compound described in the present application can be prepared into different preparation forms for topical administrations according to different affected surfaces or organs. The specific instructions are as follows:
When topically administrated to eyes, the compound described in the present application can be prepared into a micronized suspension or solution, and the carrier used is isotonic sterile saline with a certain pH, wherein a preservative such as chloride benzyl alkoxide may be added or not. In addition, for ophthalmic use, the compound can also be prepared into an ointment form such as vaseline ointment.

When topically administered to skin, the compound according to the present invention may be prepared in a suitable form of an ointment, a lotion or a cream, wherein the active ingredient is suspended or dissolved in one or more carriers. The carriers for use in an ointment preparation include, but are not limited to: mineral oil, liquid paraffin, white vaseline, propylene glycol, polyethylene oxide, polypropylene oxide, emulsion wax and water; carriers for use in a lotion or a cream include, but are not limited to: mineral oil, sorbitan monostearate, Tween 60, hexadecylester wax, hexadecane aromatic alcohol, 2-octyl dodecanol, benzyl alcohol and water.

When topically administered to lower intestine, the compound according to the present invention may be prepared in a form of the rectal suppository as described above or a suitable enema preparation, and may also be prepared in a form of a topical transdermal patch.

The compound described in the present application can also be administered in a form of sterile injection preparations, including sterile injection water or oil suspensions, or sterile injection solutions. The carriers and solvents for use therein include water, Ringer's solution, and isotonic sodium chloride solution. In addition, a sterile fixed oil can also be used as a solvent or a suspension medium, such as monoglyceride or diglyceride.

As described herein, "therapeutically effective amount" refers to an amount that is sufficient to treat or prevent a disease of patients but is low enough to avoid serious side effects (at a reasonable benefit/risk ratio) within a reasonable range of medical judgment. The therapeutically effective amount of compound will depend on the specifically selected compound (for example, considering the efficacy, effectiveness and half-life of the compound), the selected route of administration, the disease to be treated, the severity of the disease to be treated, and the factors of the patient to be treated, such as age, size, weight and physical disease, medical history, duration of treatment, nature of concurrent therapy, desired therapeutic effect and the like, but it can still be routinely determined by those skilled in the art.

In addition, it should be pointed out that the specific dosage and usage of the compound described in the present application for different patients are determined by many factors, including the patient's age, weight, gender, natural health status, nutritional status, active strength of the compound, time of administration, and metabolic rate, severity of disease and the subjective judgment of the physician who makes diagnosis and gives treatment. The preferred dose here is between 0.01 and 100 mg/kg body weight/day.

### Specific Models for Carrying Out the Application

The embodiments of the present application will be described in detail below in combination with examples, but those skilled in the art will understand that the following examples are only used to illustrate the present application and should not be regarded as limiting the scope of the present application. If specific conditions are not indicated in the examples, it shall be carried out in accordance with the conventional conditions or the conditions recommended by the manufacturer. The reagents or instruments used without the manufacturer's indication are all conventional products that can be purchased commercially.

### Example 1

Preparation of 3,7-dimethyl-oct-2,6-dienoic acid [2-(adamantan-2-yl-amino)ethyl]amide (XHJ-2-32)
1) Synthesis of N-adamantan-2-yl-ethane-1,2-diamine 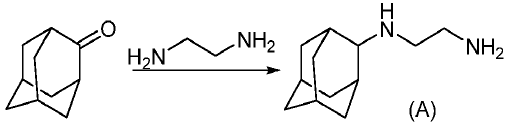 22ml (0.32mol) of Ethylenediamine was dissolved in 30ml of anhydrous methanol, placed in an ice bath and stirred to dissolve until clear, 2.0g (0.013mol) of 2-adamantanone was added under the protection of nitrogen to obtain a transparent and clear solution, which was stirred and reacted for 2h, then 0.75g (0.019mol) of NaBH₄ was slowly added in batches, and then stirred overnight at room temperature. After the reaction was completed, 50ml of H₂O was added into the reaction flask, then the resulting mixture was extracted with dichloromethane (100ml×2), the extracting solution was washed with saturated brine (50ml×2), dried with anhydrous Na₂SO₄, and the solvent was evaporated under reduced pressure to obtain 2.4g of a colorless oil with a yield of 93%. After standing, the oil was solidified to obtain a white solid. ¹HNMR (400MHz, CDCl₃), δ: 2.790~2.820(t, 2H), 2.714(s, 1H), 2.650∼2.679(t, 2H), 1. 953~1.983(d, 2H), 1.839~1.860(d, 6H), 1.691~1.713 (m, 4H), 1.480~1.509 (d, 2H), 1.424 (s, 3H).
2) Synthesis of 3,7-dimethyl-oct-2,6-dienoic acid [2-(adamantan-2-yl-amino)ethyl]amide (XHJ-2-32) 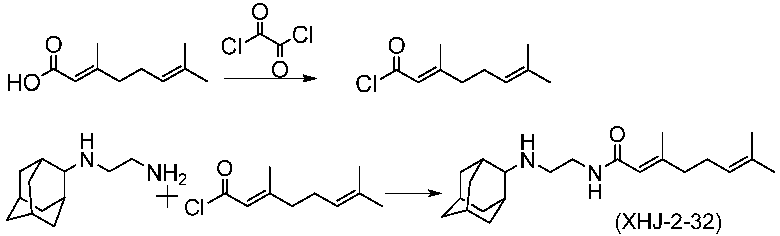 3,7-Dimethyl-oct-2,6-dienylacyl chloride was obtained through the acylation reaction of 3,7-dimethyl-oct-2,6-dienoic acid with oxalyl chloride, in which 1.5 times the amount of oxalyl chloride was added dropwise to 3,7-dimethyl-oct-2,6-dienoic acid, after the reaction was completed, the reaction liquid was distilled off and the obtained solid could be directly used in the next reaction.

0.35g of N-adamantan-2-yl-ethane-1,2-diamine was added into a reaction flask, 10ml of dry THF was added to dissolve the N-adamantan-2-yl-ethane-1,2-diamine, 0.6g of potassium carbonate was added, then 0.3g of 3,7-dimethyl-oct-2,6-dienylacyl chloride (dissolved in 5ml of dry THF) was added slowly and dropwise under ice bath. After the addition was completed, the reaction solution turned from colorless to reddish-brown, and reacted under stirring until the reaction of 3,7-dimethyl-oct-2,6-dienylacyl chloride was completed, 50ml of H₂O was added into the reaction flask, then the resulting mixture was extracted with dichloromethane (100ml×2), the extracting solution was washed with saturated brine (50ml×2), dried with anhydrous Na₂SO₄, concentrated under reduced pressure, separated and purified by a silica gel column (eluent was the mixture of dichloromethane, methanol and ammonia, wherein dichloromethane : methanol : ammonia (v/v/v)=500:10:1) to obtain 154mg of a pale yellow oil with a yield of 28%. The oil was salified by treatment with hydrogen chloride in ethyl ether to obtain a white solid, mp 150-164°C. ¹HNMR (400MHz, CDCl₃), δ: 5.645(s, 1H), 5.074(t, 1H), 3.572~3.584(t, 2H), 2.964~2.996(d, 3H), 2.110~2.155(m, 10H), 1.881 (t, 4H), 1.682~1.734(t, 7H), 1.568~1.605(d, 5H). LC-MS, m/z(%): 345.3(M⁺)

### Example 2

### Preparation of N-(adamantan-2-yl)-N'-ethylsulfonyl-ethane-1,2-diamine (XHJ-3-46)

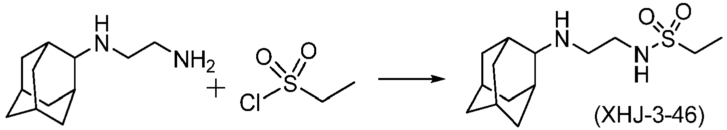

The synthesis steps of Example 1 were repeated, except that ethylsulfonyl chloride was used as a raw material in place of 3,7-dimethyl-oct-2,6-dienylacyl chloride, and the target product XHJ-3-46 was obtained after separation and purification, yield: 28%, Mp 172-174°C. ¹HNMR (400MHz, CDCl₃), δ: 3.17~3.19 (t, 2H), 3.04~3.10(m, 2H), 2.82~2.85(t, 2H), 2. 72 (s, 1H), 1.80~1.93(m, 8H), 1.68~1.72(d, 2H), 1.51~1.54(d, 2H), 1.36~1.40(t, 3H). LC-MS, m/z(%): 287.3(M⁺)

### Example 3

### Preparation of N-(adamantan-2-yl)-N'-octyl-ethane-1,2-diamine dihydrochloride (XHJ-5-3)

The synthesis steps of Example 1 were repeated, except that 1-bromooctane was used as a raw material in place of 3,7-dimethyl-oct-2,6-dienylacyl chloride, petroleum ether was used in place of dichloromethane during the extraction in post-treatment, a colorless oil N-(adamantan-2-yl)-N'-octyl-ethane-1,2-diamine was obtained after separation and purification, which was salified by treatment with hydrogen chloride in ethyl ether to obtain the target product XHJ-5-3, yield: 15.2%, mp 224-226°C. ¹HNMR (400MHz, CDCl₃), δ: 3.44~3.48 (m, 5H), 3.06~3.10(t, 2H), 2.24 (s, 2H), 2.10~2.13 (d, 2H), 2.00~2.03(d, 2H), 1.83~1.93(m, 6H), 1.72~1.78(m, 4H), 1.32~1.41(m, 10H), 0.88~0.92(t, 3H). LC-MS, m/z(%):307.4(M⁺)

### Example 4

### Preparation of N-(adamantan-2-yl)-N'-hexyl-ethane-1,2-diamine dihydrochloride (XHJ-5-5)

The synthesis steps of Example 3 were repeated, except that 1-bromohexane was used as a raw material in place of 1-bromooctane, after separation and purification, the product was salified by treatment with hydrogen chloride in ethyl ether to obtain the target product XHJ-5-5, yield: 18.0%, mp 212-216°C. ¹HNMR (400MHz, CDCl₃), δ: 3.43~3.48 (m, 5H), 3.05~3. 09(t, 2H), 2.22 (s, 2H), 2.08~2.11 (d, 2H), 1.99~2.02(d, 2H), 1.93(s, 2H), 1.83~1.87(m, 4H), 1.71~1.77(m, 4H), 1.33~1.43(m, 6H), 0.90~0.94(t, 3H). LC-MS, m/z(%):279.4(M⁺)

### Example 5

### Preparation of N-(adamantan-2-yl)-N'-cyclopropyl-ethane-1,2-diamine (XHJ-5-18)

The synthesis steps of Example 3 were repeated, except that bromocyclopropane was used as a raw material in place of 1-bromooctane, after separation and purification, the target product XHJ-5-18 was obtained, yield: 37.4%, mp197-200°C. ¹HNMR(400MHz, CDCl₃),δ: 2.94~3.16 (m, 3H), 2.77~2. 78(d, 2H), 2.12~2.15 (d, 2H), 1.99(s, 2H), 1.68~1.83(m, 8H), 1.43~1.47(d, 2H), 1.07~1.09(m, 1H), 0.54~0.55(d, 2H), 0.35~0.38(t, 2H). LC-MS, m/z(%):234.2(M⁺)

### Example 6

### Preparation of N,N-(adamantan-2-yl)-(3,7-dimethyl-oct-2,6-dienyl)-ethane-1,2-diamine (XHJ-3-45)

The synthesis steps of Example 3 were repeated, except that geranyl chloride was used as a raw material in place of 1-bromooctane, the separated product contained positive drug SQ109 and target product XHJ-3-45, mp102-104°C. ¹HNMR(400MHz, CDCl₃),δ: 5.30~5.34 (t, 1H), 5.04~5. 07(t, 1H), 3.48~3.50 (d, 2H), 3.11(s, 4H), 3.00(s, 1H), 2.01~2.09(m, 8H), 1.89~1.92(t, 4H), 1.68~1.77(m, 10H), 1.60~1.64(m, 3H). LC-MS, m/z(%):331.6(M⁺)

### Example 7

### Preparation of N-geranyl-N-(2-aminoethyl)ethylsulfonylamide hydrochloride (XHJ-2-39)

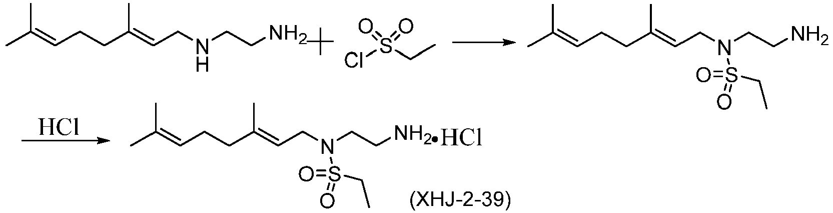

1.06g of N-geranyl-ethane-1,2-diamine was added into a reaction flask, 20ml of dry THF and 0.8ml of triethylamine were added, then 0.73g of ethylsulfonyl chloride (dissolved in 10ml) was added dropwise, after the addition was completed, the reaction was performed under stirring at room temperature until the reaction was completed, then 50ml of H₂O was added into the reaction flask, the resulting mixture was extracted with ethyl acetate (50ml×3), the extracting solution was washed with saturated brine (50ml×2), dried overnight with anhydrous Na₂SO₄, filtered under reduced pressure, and evaporated to remove the solvent to obtain 1.5g of a yellow oil, the yellow oil was separated and purified by a silica gel column (eluent was the mixture of dichloromethane, methanol and ammonia, wherein dichloromethane : methanol : ammonia (v/v/v)=500:10:1) to obtain 420mg of a pale yellow oil with a yield of 26.9%, which was salified by treatment with hydrogen chloride in ethyl ether to obtain a white solid, mp103-106°C. ¹HNMR(400MHz, CDCl₃),δ: 5.189~5.223 (t, 1H), 5.067~5.098(t, 1H), 3.217~3.234 (d, 2H), 3.166~3.193(t, 2H), 3.031~3.087(m, 2H), 2.789~2.817(t, 2H), 2.062~2.098(m, 2H), 2.012~2.032(t, 2H), 1.605~1.682(t, 9H), 1.357~1.394(t, 5H). LC-MS, m/z(%):289.3 (M+). LC-MS, m/z(%):289.3(M⁺)

### Example 8

### Preparation of N-geranyl-N-(2-aminoethyl)-p-toluenesulfonamide hydrochloride (XHJ-2-45)

The synthesis steps of Example 7 were repeated, except that p-toluenesulfonyl chloride was used as a raw material in place of ethylsulfonyl chloride, a pale yellow oil N-geranyl-N-(2-aminoethyl)-p-toluenesulfonamide was obtained after separation and purification, which was salified by treatment with hydrogen chloride in ethyl ether to obtain the target product XHJ-2-45 with a yield of 18.2%, mp 134-137°C. ¹HNMR(400MHz, CDCl3),δ: 7.661~7.682 (d, 2H), 7.380~7. 399(d, 2H), 5.058~5.072 (t, 2H), 5.067~5.098(m, 1H), 2.983~3.000 (d, 2H), 2.749~2.782(t, 2H), 2.461~2.477(t, 2H), 2.380(s, 3H), 1.996~2.031(m, 2H), 1.906~1.943t, 2H), 1.633(s, 3H), 1.531~1.558(d, 6H). LC-MS, m/z(%):351.3(M⁺)

### Example 9

### Preparation of N-(dipyridin-2-yl-methyl)-N'-(3,7-dimethyl-oct-2,6-dienyl)-ethane-1,2-diamine (XHJ-5-14)

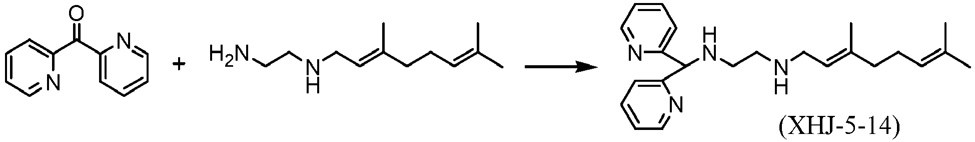

1.0g (5mmol) of N-geranyl-ethane-1,2-diamine was added into a reaction flask, 20ml of anhydrous methanol was added to dissolve the N-geranyl-ethane-1,2-diamine, 1.1g (5.9mmol) of di-2-pyridyl ketone was added, the resulting reaction solution was reacted under stirring and N₂ protection at room temperature for 4h, then 0.3g (7.9mmol) of NaBH₄ was slowly added in batches, and then the reaction solution was stirred at room temperature overnight. After the reaction was completed, 100ml of H₂O was added into the reaction flask, the reaction solution was extracted with petroleum ether (100ml×3), the organic layer was washed with saturated brine (50ml×2), dried with anhydrous Na₂SO₄, distilled under reduced pressure to remove the solvent and obtain 2.03g of a pale yellow oil, which was subjected to column chromatography (CH₂Cl₂:CH₃OH:NH₃•H₂O(v/v/v)=500:10:1) to obtain 0.36g of a pale yellow oil with a yield of 19.7%. ¹HNMR(400MHz, CDCl3),δ: 8.56~8.57 (d, 2H), 7.60~7. 62(t, 2H), 7.37~7.39 (d, 2H), 7.13~7.16 (m, 2H), 5.28~5.29(t, 1H), 5.06~5.08(t, 2H), 3.24~3.28(t, 2H), 2.81~2.84(m, 4H), 1.99~2.07(m, 4H), 1.59~1.72(m, 9H), 1.633(s, 3H), 1.531~1.558(d, 6H). LC-MS, m/z(%):365.3(M⁺)

### Example 10

### Preparation of N-(phenyl-pyridin-3-yl-methyl)-N'-(3,7-dimethyl-oct-2,6-dienyl)-ethane- 1,2-diamine (XHJ-5-8)

The synthesis steps of Example 9 were repeated, except that 3-benzoylpyridine was used as a raw material in place of di-2-pyridyl ketone, and sodium cyanoborohydride was used in place of sodium borohydride, and the target compound XHJ-5-8 was obtained after separation and purification. ¹HNMR(400MHz, CDCl₃),δ: 8.51 (d, 1H), 8.29~8. 30(m, 1H), 7.80~7.82 (m, 1H), 7.23~7.36 (m, 5H), 7.15~7.17 (m, 1H), 5.13~5.15(t, 1H), 5.01~5.03(t, 1H), 3.11~3.13(d, 2H), 2.59~2.65(m, 4H), 1.94~2.03(m, 5H), 1.48~1.60(m, 10H), 1.19~1.20(d, 1H), 1.10~1.13(t, 1H). LC-MS, m/z(%):364.2(M⁺)

### Example 11

### Preparation of N-adamantan-2-yl-N',N'-ethylsulfonyl-(3,7-dimethyl-oct-2,6-dienyl)-ethane-1,2-diamine (XHJ-3-1)

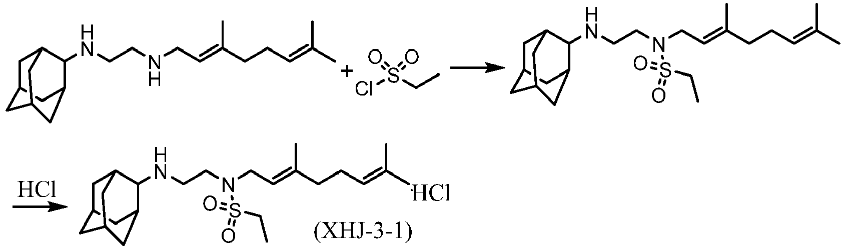

0.9g (2.72mmol) of SQ109 (N-adamantan-2-yl-N'-(3,7-dimethyl-oct-2,6-dienyl)-ethane-1,2-diamine) was dissolved in 15ml of dry THF, 0.6g of potassium carbonate was added, and 0.35g (2.7mmol) of ethylsulfonyl chloride (dissolved in 5ml of THF) was added dropwise at room temperature. After the dropwise addition was completed, the temperature was raised and the reaction was performed under refluxing for 4h. After the reaction was completed, 50ml of H₂O was added into the reaction flask, the resulting mixture was extracted with petroleum ether (100ml×2), the organic layer was washed with saturated brine (50ml×2), dried overnight with anhydrous Na₂SO₄, filtered under reduced pressure, and evaporated to remove the solvent to obtain 1.0g of a yellow liquid, which was separated and purified by a silica gel column (the eluent was the mixture of dichloromethane, methanol and ammonia; wherein dichloromethane : methanol : ammonia(v/v/v) =1000:10:1) to obtain 240 mg of a pale yellow oil, with a yield of 30.5%, which was salified by treatment with hydrogen chloride in ethyl ether to obtain a white solid, mp103-106°C. ¹HNMR(400MHz, CDCl₃),δ: 5.202~5.235(t, 1H), 5.042~5.059(t, 1H), 3.907~3.925(d, 2H), 3.302~3.334(t, 2H), 3.011~3.067(m, 2H), 2.760~2.791(t, 2H), 2.705(t, 1H), 2.045~2.106(m, 4H), 1.930~1.961(d, 2H), 1.833~1.851(t, 6H), 1.603~1.706(m, 10H), 1.472~1.502(d, 2H), 1.336~1.373(t, 3H).LC-MS, m/z(%):423.4(M⁺)

### Example 12

### Preparation of N-adamantan-2-yl-N',N'-p-toluenesulfonyl-(3,7-dimethyl-oct-2,6-dienyl)-ethane-1,2-diamine (XHJ-3-3)

The synthesis steps of Example 11 were repeated, except that p-toluenesulfonyl chloride was used as a raw material in place of ethylsulfonyl chloride, a pale yellow oil was obtained after separation and purification, which was salified by treatment with hydrogen chloride in ethyl ether to obtain a white solid, namely the target compound XHJ-3-3, mp179-182°C. ¹HNMR(400MHz, CDCl₃),δ: 7.706~7.727(d, 2H), 7.266~7.297(t, 2H), 5.003~5.010(t, 2H), 3.843~3.860(d, 2H), 3.189~3.222(t, 2H), 2.734~2.767(t, 2H), 2.666(s, 1H), 2.417(s, 3H), 1.912~2.025(m, 6H), 1.762~1.841(m, 6H), 1.668~1.698(d, 7H), 1.575~1.610(d, 6H), 1.452~1.483(d, 2H). LC-MS, m/z(%):485.6(M⁺)

### Example 13

### Preparation of N-adamantan-2-yl-N',N'-(3,7-dimethyl-oct-2,6-dienyl)-(3,7-dimethyl-oct-2,6-dienoyl)-ethane-1,2-diamine (XHJ-3-11)

The synthesis steps of Example 11 were repeated, except that 3,7-dimethyl-oct-2,6-dienoyl chloride was used as a raw material in place of ethylsulfonyl chloride, and a pale yellow oil was obtained after separation and purification, which was the target compound XHJ-3-11. ¹HNMR(400MHz, CDCl₃),δ: 5.779(s, 1H), 5.051~5.064(t, 3H), 3.959~3.975(d, 2H), 3.645~3.655(t, 2H), 3.093(s, 2H), 1.899~2.146(t, 19H), 1.572~1.745(m, 21H), 1.202~1.226(d, 1H). LC-MS, m/z(%):481.6(M+).

### Example 14

### Preparation of N-adamantan-2-yl-N',N'-(3-indolyl-propionyl)-(3,7-dimethyl-oct-2,6-dienyl)-ethane-1,2-diamine (XHJ-3-27)

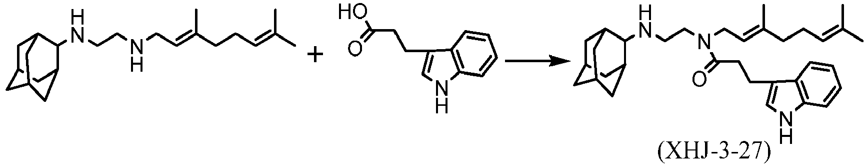

0.4g of 3-indolyl-propionic acid, 0.35g of HOBT (1-hydroxybenzotriazole), and 0.32g of DMAP (dimethylaminopyridine) were dissolved in 15ml of DMF, 2ml of triethylamine was added, and 0.75g of SQ109 was added, then the resulting mixture was heated to 50°C, and then 0.57 of EDCI (1-(3-dimethylaminopropyl)-3-ethylcarbodiimide hydrochloride) was added, the resulting reaction solution was reacted until it was detected from TLC that the reaction was completed. After the reaction was completed, the reaction system was allowed to stand and be cooled, 100ml of H₂O was added into the reaction flask, the reaction solution was extracted with ethyl acetate (100ml×2), the organic layer was washed with saturated brine (50ml×2), dried overnight with anhydrous Na₂SO₄, filtered under reduced pressure, and evaporated to remove the solvent and obtain 0.82g of a red-brown oil, which was separated and purified by silica gel column (the eluent was the mixture of dichloromethane, methanol and ammonia, wherein dichloromethane: methanol: ammonia (v/v/v)=1000:10:1) to obtain 110 mg of a pale yellow oil, which was frozen and solidified to obtain a white solid, with a yield of 9.4%. ¹HNMR(400MHz, DMSO-D6),δ:8.58(s, 1H), 7.57~7.62(t, 1H), 7.32~7.34(d, 1H), 7.14~7.18(t, 1H), 7.06~7.10(t, 1H), 6.98(s, 1H), 5.07~5.13(t, 1H), 4.98~5.05(t, 1H), 4.05~4.06(d, 1H), 3.83~3.85(d, 2H), 3.45~3.49(t, 1H), 3.27~3.30(t, 1H), 3.11~3.17(m, 2H), 2.65~2.81(m, 5H), 1.57~2.08(m, 25H), 1.43~1.49(t, 2H).LC-MS, m/z(%):502.6(M⁺)

### Example 15

### Preparation of N-adamantan-2-yl-N',N'-(3,4,5-trimethoxybenzoyl)-(3,7-dimethyl-oct-2,6-dienyl)-ethane-1,2-diamine (XHJ-3-29)

The synthesis steps of Example 14 were repeated, except that 3,4,5-trimethoxybenzoic acid was used as a raw material in place of 3-indolyl-propionic acid, a pale yellow oil was obtained after separation and purification, which was frozen and solidified to obtain a pale yellow solid, namely the target compound XHJ-3-29. ¹HNMR(400MHz, CDCl₃),δ:6.65(s, 2H), 5.27~5.30(t, 1H), 5.07(s, 1H), 4.18(s, 1H), 3.82~3.93(m, 10H), 3.60(s, 2H), 2.91(s, 1H), 2.74~2.77(d, 2H), 2.04~2.08(d, 5H), 1.52~1.84(m, 20H). LC-MS, m/z(%):525.1(M⁺)

### Example 16

### Preparation of N-adamantan-2-yl-N',N'-undecanoyl-(3,7-dimethyl-oct-2,6-dienyl)-ethane-1,2-diamine (XHJ-3-31)

The synthesis steps of Example 14 were repeated, except that undecanoic acid was used as a raw material in place of 3-indolyl-propionic acid, a pale yellow oil was obtained after separation and purification, which was the target compound XHJ-3-31. ¹HNMR(400MHz, CDCl₃),δ: 5.06~5.10(m, 2H), 4.02~4.03(d, 1H), 4.92~4.94(d, 1H), 3.44~3.47(t, 1H), 3.34~3.37(t, 1H), 3.72~3.77(m, 3H), 2.36~2.40(t, 1H), 2.27~2.31(t, 1H), 1.59~2.08(m, 30H), 1.25~1.29(m, 16H), 0.86~0.89(t, 3H). LC-MS, m/z(%):513.8(M⁺)

### Example 17

Preparation of N,N-adamantan-2-yl-(3-indolylformyl)-N',N'-(3,7-dimethyl-oct-2,6-dienyl)-(3-indolylformyl)-ethane-1,2-diamine (XHJ-3-15)

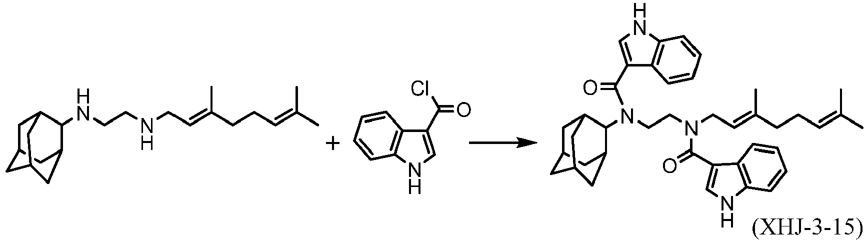

1.0g (3.0mmol) of SQ109 was dissolved in 20ml of dry THF, 0.7ml of triethylamine was added, and 1.1g (6.1mmol) of 3-indolylformyl chloride (dissolved in 5ml of THF) was added dropwise under ice bath; after the addition was completed, the reaction system was heated and refluxed for 2h. After the reaction was completed, 50ml of H₂O was added to the reaction flask, the resulting mixture was extracted with dichloromethane (100ml×2), the organic layer was washed with saturated brine (50ml×2), dried overnight with anhydrous Na₂SO₄, filtered under reduced pressure, and evaporated to remove the solvent to obtain a yellow solid, which was recrystallized in methanol to obtain 1.12g of a white solid, with a yield of 59.9%. mp248-252°C. ¹HNMR(400MHz, DMSO-D6),δ:11.61(s, 1H), 11.48(s, 1H), 7.73(s, 1H), 7.40~7.62(m, 5H), 7.03~7.16(m, 4H), 4.96~4.99(t, 1H), 4.85~4.88(t, 1H), 3.73(s, 4H), 1.54~1.92(m, 21H), 1.16(s, 3H). LC-MS, m/z(%):618.8(M⁺).

### Example 18

### Preparation of N,N-adamantan-2-yl-benzyl-N',N'-(3,7-dimethyl-oct-2,6-dienyl)-benzyl-ethane-1,2-diamine (XHJ-4-11)

The synthesis steps of Example 17 were repeated, except that benzyl bromide was used as a raw material in place of 3-indolylformyl chloride, and a pale yellow oil was obtained by separation and purification, which was frozen and solidified to obtain the target compound XHJ-4-11. ¹HNMR(400MHz, CDCl₃),δ: 7.22~7.29(m, 10H), 5.17~5.20(t, 1H), 5.06~5.10(t, 1H), 3.62(s, 2H), 3.40(s, 2H), 2.89~3.91(d, 2H), 2.63~2.67(t, 2H), 2.37~2.41(t, 2H), 1.94~2.12(m, 8H), 1.79~1.81(t, 4H), 1.66~1.71(t, 6H), 1.60~1.63(d, 5H), 1.51~1.53(t, 3H), 1.35~1.38(d, 2H). LC-MS, m/z(%):511.8(M⁺)

### Example 19

### Preparation of N,N-adamantan-2-yl-octyl-N',N'-(3,7-dimethyl-oct-2,6-dienyl)-octyl- ethane-1,2-diamine (XHJ-4-15)

The synthesis steps of Example 17 were repeated, except that 1-bromooctane was used as a raw material in place of 3-indolylformyl chloride, and a pale yellow oil was obtained by separation and purification, which was the target compound XHJ-4-15. ¹HNMR(400MHz, CDCl₃),δ: 5.24~5.25(t, 1H), 5.09~5.10(t, 1H), 3.05~3.06(d, 2H), 2.63(s, 2H), 2.40~2.52(d, 2H), 1.95~2.06(m, 8H), 1.60~1.80(m, 16H), 1.27~1.40(m, 25H), 0.86~0.90(t, 6H). LC-MS, m/z(%):555.7(M⁺)

### Example 20

### Preparation of N-adamantan-2-yl-2-(3,7-dimethyl-oct-2,6-dienylamino)-acetamide hydrochloride (XHJ-2-37)

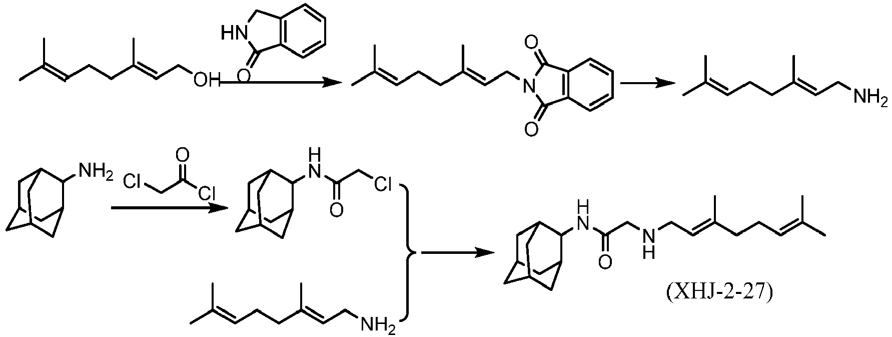

### 1) Synthesis of geranylamine

2.51g (16.2mmol) of geraniol, 2.86g (19.4mmol) of phthalimide, and 5.12g (19.5mmol) of triphenylphosphine were dissolved in 50ml of dry THF, 3.35g (19.2mmol) of DEAD (diethyl azodicarboxylate) was added dropwise under ice bath. After the addition was completed, the resulting reaction system was stirred and reacted at room temperature until the reaction was completed, then evaporated to remove the solvent and obtain a white solid, which was extracted with petroleum ether, separated and purified by a silica gel column (the eluent was the mixture of petroleum ether and ethyl acetate, wherein petroleum ether : ethyl acetate (v/v) = 10:1) to obtain 3.0g of an intermediate 2-(3,7,-dimethyl-oct-2,6-dienyl)-isoindole-1,3-dione, with a yield of 65.4%. ¹HNMR(400MHz, CDCl₃),δ: 7.817~7.848(m, 2H), 7.699~7.713(m, 2H), 5.249~5.282(t, 1H), 5.024~5.058(t, 1H), 4.272~4.289(d, 2H), 1.990~2.075(m, 4H), 1.830(s, 3H), 1.631(s, 3H), 1.566(s, 3H).

3.0g (10.6mmol) of 2-(3,7,-dimethyl-oct-2,6-dienyl)-isoindole-1,3-dione was dissolved in 70ml of absolute ethanol, and 5ml (85mmol) of hydrazine hydrate was added, the resulting mixture was heated to 70°C and reacted for 3h. After the reaction was completed, 70ml of H₂O was added into the reaction flask, then the reaction solution was adjusted with concentrated hydrochloric acid to a pH of 2, washed with 80ml of ethyl ether. The aqueous phase was adjusted with 2M sodium hydroxide solution to a pH of 12, and extracted with anhydrous ethyl ether (100×2)^{[48]}, the extracting solution was washed with saturated brine (50ml×2), dried overnight with anhydrous Na₂SO₄, filtered under reduced pressure, and evaporated to remove the solvent to obtain 1.0 g of a yellow liquid with a yield of 61.7%. ¹HNMR(400MHz, CDCl₃),δ: 5.243~5.274(t, 1H), 5.082~5.099(t, 1H), 3.269~3.286(d, 2H), 1.978~2.117(m, 4H), 1.606~1.685(m, 8H), 1.442(s, 3H).

### 2) Synthesis of N-adamantan-2-yl-2-(3,7-dimethyl-oct-2,6-dienylamino)-acetamide hydrochloride

0.11g (0.48mmol) of N-adamantyl-acetyl chloride was dissolved in 20ml of dry THF, 0.3g of potassium carbonate was added, and 0.63g (4.11mmol) of geranylamine was added, the resulting mixture was heated and reacted under refluxing for 4h. After the reaction was completed, 30ml of H₂O was added into the reaction flask, the reaction solution was extracted with dichloromethane (50ml×2), the organic layer was washed with saturated brine (30ml×2), dried overnight with anhydrous Na₂SO₄, filtered under reduced pressure, and evaporated to remove the solvent to obtain 0.55g of a red-brown liquid, which was separated and purified by silica gel column (the eluent was the mixture of dichloromethane, methanol and ammonia, wherein dichloromethane : methanol : ammonia (v/v/v)=500:10:1) to obtain 42mg of a pale yellow oil with a yield of 26.2%, which was salified by treatment with hydrogen chloride in ethyl ether to obtain a white solid. ¹HNMR(400MHz, DMS0-D6),δ: 7.825~7.845(t, 1H), 5.208~5.243(t, 1H), 5.064~5.096(t, 1H), 4.061~4.082(d, 1H), 3.245~3.391(m, 3H), 1.997~2.096(m, 4H), 1.816~1.906(t, 10H), 1.750(s, 2H), 1.593~1.684(m, 12H).LC-MS, m/z(%):345.3(M⁺)

Example 21: In vitro anti-tuberculosis activity test of the compound of the present invention The Microplate Alamar Blue Assay (MABA) method was used to determine the minimum inhibitory concentration (MIC) of the compound against the standard strain H₃₇Rv of *Mycobacterium tuberculosis* (see Collins LA, Franzblau S G., Microplate alamar blue assay versus BACTEC 460 system for high-throughput screening of compounds against *Mycobacterium tuberculosis* and *Mycobacterium avium.* Antimicrobial Agents Chemother, 1997, 1004-1009). The compounds to be tested were the compounds prepared in the Examples 1 to 20 of the present application, and the positive control compounds rifampin (RFP) and isoniazid (INH).

Experimental method: sterile 96-well plates (Falcon3072; Becton Dickinson, Lincoln Park, NJ) were used, the compound to be tested was dissolved in dimethyl sulfoxide to prepare an initial solution with a concentration of 5mg/ml, 199µl of 7H9 liquid culture medium (Difco) and 1µl of the initial solution of the compound was added to the highest concentration well and mixed well; the concentrations of the remaining wells were gradiently reduced twice by dilution, in which the volume of the solution of the compound to be tested was 200µl and the final concentrations of the test compound in each well were: 25, 12.5, 6.25, 3.125, 1.56, 0.78, 0.39, 0.2, 0.1, 0.05, 0.025, 0.0125 µg/ml. *Mycobacterium tuberculosis* H₃₇Rv (preserved strain of Beijing Tuberculosis and Thoracic Tumor Research Institute) was cultured for 2 to 3 weeks to prepare a bacterial suspension, inoculated into 7H9 liquid medium containing 0.05% Tween 80 and 10% ADC (albumin-dextrose-catalase, ADC), subjected to static culture at 37°C for 1 to 2 weeks; when growing to a turbidity of McFarland 1 (equivalent to 10⁷CFU/ml), the dilution was performed by a volume ratio of 1:20, then 100µl of bacterial suspension was taken and added to each well. The final concentration of bacterial solution in each well was about 10⁶CFU/ml. For each plate, two growth control wells without antibiotics were set, the 96-well plates were incubated at 37°C. After 7 days, a mixture solution of 20µl of 10×Alamar Blue (Setotec) and 50µl of 5% Tween80 was added to the growth control wells, then incubation was performed at 37°C for 24 hours. If the color changed from blue to pink, the above-mentioned amount of the mixture solution of Alamar Blue and Tween80 was added into the wells of each drug to be tested, and incubated at 37°C for 24 hours, the color of each well was recorded, the fluorescence values of 530nm and 590nm was measured by a microplate reader, and MIC values were calculated. The results are shown in Table 1. MIC was defined as the lowest drug concentration that prevented the color change (from blue to pink).

**Table 1: Results of minimum inhibitory concentration (MIC) of Mycobacterium tuberculosis standard strain H37Rv determined by Microplate Alamar Blue Assay (MABA) method**

| Sample No. | MIC (µg/ml) |
|---|---|
| XHJ-2-37 | <0.5 |
| XHJ-2-32 | 7.754 |
| XHJ-2-39 | >32 |
| XHJ-2-45 | 31.459 |
| XHJ-3-1 | 6.961 |
| XHJ-3-3 | 3.757 |
| XHJ-3-11 | >32 |
| XHJ-3-15 | >32 |
| XHJ-3-27 | 1.963 |
| XHJ-3-29 | 3.91 |
| XHJ-3-31 | 2.844 |
| XHJ-3-45 | <0.5 |
| XHJ-3-46 | >32 |
| XHJ-4-11 | 30.822 |
| XHJ-4-15 | >32 |
| XHJ-5-3 | 0.986 |
| XHJ-5-5 | 0.982 |
| XHJ-5-8 | 16 |
| XHJ-5-14 | 4 |
| XHJ-5-18 | >32 |
| INH | 0.05 |
| RFP | 0.048 |

It can be seen from the experimental data in Table 1 that the compounds of the present invention had good in vitro anti-tuberculosis activity. Under the premise of retaining the basic skeleton structure of SQ109, among the modifications of its structure, the linear amides showed good activity, and both mono-substituted amides and sulfonamides at the N position showed good activity, and such examples included XHJ-2-37, XHJ-2-32, XHJ-3-1, XHJ-3-3, XHJ-3-27, XHJ - 3-29, XHJ-3-31. Among the isopentenyl substitution derivatives, when the isopentenyl was replaced with a linear alkyl, better activity was observed, and such examples included XHJ-5-3 and XHJ-5-5. Among the adamantyl substitution derivatives, XHJ-5-8 and XHJ-5-14 showed better activity, and the skeleton rearrangement derivative XHJ-3-45 had better activity.

## Claims

1. A compound represented by Formula A, a pharmaceutically acceptable salt, a stereoisomer, a tautomer or an isomer mixture, a hydrate, a solvate or a prodrug thereof, 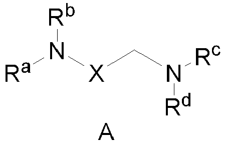
wherein, R^{a} is hydrogen, C₁₋₆ alkyl, C₃₋₁₄ cycloalkyl, 6- to 14-membered aryl, 3- to 14-membered heterocyclyl, 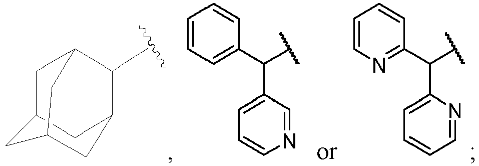
R^{b} is hydrogen, C₁₋₁₀ alkyl, C₃₋₁₄ cycloalkyl, C₂₋₁₂ alkenyl or polyenyl, C₂₋₁₂ alkenylacyl or polyenylacyl, C₂₋₁₀ alkylacyl, substituted or unsubstituted 6- to 14-membered aryl, substituted or unsubstituted 3- to 14-membered heterocyclyl, substituted or unsubstituted 7- to 12-membered bridged-ring group, phenyl-substituted C₁₋₆ alkyl or indol-3-yl-(CH₂)ₙ-C(O)-, wherein n is an integer between 0 and 4;
X is -CH₂-, -CHR₂- or -C(O)-, wherein R₂ is C₁₋₆ alkyl;
R^{c} is hydrogen, C₁₋₁₀ alkyl, C₃₋₁₄ cycloalkyl, C₂₋₁₀ alkylacyl, C₂₋₁₂ alkenyl or polyenyl, C₂₋₁₂ alkenylacyl or polyenylacyl, substituted or unsubstituted sulfonyl, substituted or unsubstituted 6-to 14-membered aryl, substituted or unsubstituted 3- to 14-membered heterocyclyl, substituted or unsubstituted 7- to 12-membered bridged-ring group;
R^{d} is hydrogen, C₁₋₁₀ alkyl, C₃₋₁₄ cycloalkyl, C₂₋₁₂ alkenyl or polyenyl, C₂₋₁₂ alkenylacyl or polyenylacyl, C₂₋₁₂ alkylacyl, substituted or unsubstituted 6- to 14-membered aryl, substituted or unsubstituted 3- to 14-membered heterocyclyl, substituted or unsubstituted 7- to 12-membered bridged-ring group, phenyl-substituted C₁₋₁₀ alkyl, substituted or unsubstituted sulfonyl, substituted or unsubstituted benzoyl, indol-3-yl-(CH₂)ₙ-C(O)-, wherein n is an integer between 0 and 4,
for example, R^{a} is hydrogen, 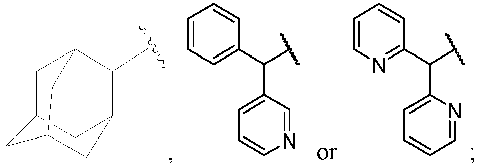
for example, R^{b} is hydrogen, 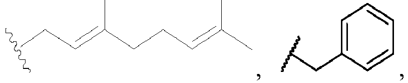
- CH₂(CH₂)₆CH₃, or 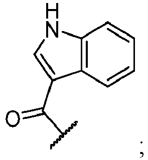
X is -CH₂- or -C(O)-;
for example, R^{c} is hydrogen, 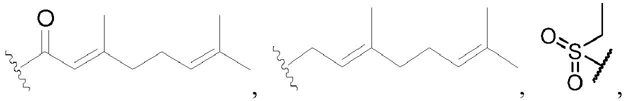 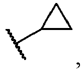
- CH₂(CH₂)₆CH₃, or -CH₂(CH₂)₄CH₃;
for example, R^{d} is hydrogen, 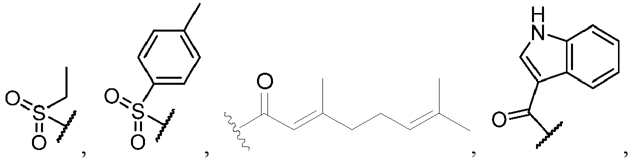 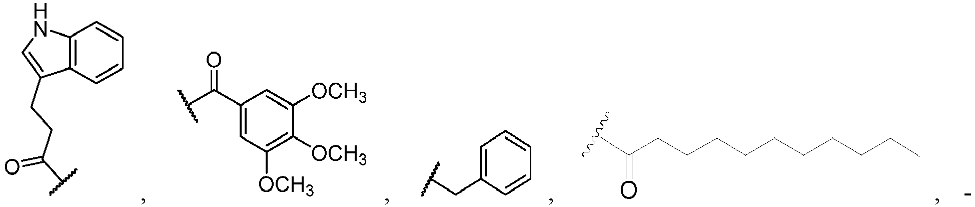 CH2(CH2)6CH3.

2. The compound, a pharmaceutically acceptable salt, a stereoisomer, a tautomer or an isomer mixture, a hydrate, a solvate or a prodrug thereof according to claim 1, wherein the compound is represented by Formula I, 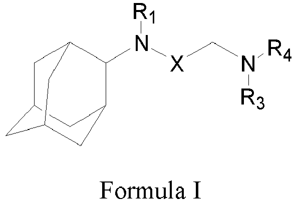 wherein,
R₁ is hydrogen, C₁₋₁₀ alkyl, C₃₋₁₄ cycloalkyl, C₂₋₁₂ alkenyl or polyenyl, C₂₋₁₂ alkenylacyl or polyenylacyl, C₂₋₁₀ alkylacyl, substituted or unsubstituted 6- to 14-membered aryl, substituted or unsubstituted 3- to 14-membered heterocyclyl, substituted or unsubstituted 7- to 12-membered bridged-ring group, phenyl-substituted C₁₋₆ alkyl or indol-3-yl-(CH₂)ₙ-C(O)-, wherein n is an integer between 0 and 4;
X is -CH₂-, -CHR₂- or -C(O)-, wherein R₂ is C₁₋₆ alkyl;
R₃ is hydrogen, C₁₋₁₀ alkyl, C₃₋₁₄ cycloalkyl, C₂₋₁₂ alkenyl or polyenyl, C₂₋₁₂ alkenylacyl or polyenylacyl, C₂₋₁₀ alkylacyl, substituted or unsubstituted 6- to 14-membered aryl, substituted or unsubstituted 3- to 14-membered heterocyclyl, substituted or unsubstituted 7- to 12-membered bridged-ring group, phenyl-substituted C₁₋₁₀ alkyl, substituted or unsubstituted sulfonyl, substituted or unsubstituted benzoyl, indol-3-yl-(CH₂)ₙ-C(O)-, wherein n is an integer between 0 and 4;
R₄ is hydrogen, C₁₋₁₀ alkyl, C₃₋₁₄ cycloalkyl, C₂₋₁₀ alkylacyl, C₂₋₁₂ alkenyl or polyenyl, C₂₋₁₂ alkenylacyl or polyenylacyl, substituted or unsubstituted sulfonyl, substituted or unsubstituted 6-to 14-membered aryl, substituted or unsubstituted 3- to 14-membered heterocyclyl, substituted or unsubstituted 7- to 12-membered bridged-ring group.

3. The compound, a pharmaceutically acceptable salt, a stereoisomer, a tautomer or an isomer mixture, a hydrate, a solvate or a prodrug thereof according to claim 2,
wherein, R₁ is hydrogen, C₁₋₁₀ alkyl, C₃₋₁₄ cycloalkyl, C₂₋₁₂ alkenyl or polyenyl, C₂₋₁₂ alkenylacyl or polyenylacyl, C₂₋₁₂ alkylacyl, substituted or unsubstituted 6- to 14-membered aryl, substituted or unsubstituted 3- to 14-membered heterocyclyl, substituted or unsubstituted 7- to 12-membered bridged-ring group;
R₃ is hydrogen, C₁₋₁₀ alkyl, C₃₋₁₄ cycloalkyl, C₂₋₁₂ alkenyl or polyenyl, C₂₋₁₂ alkenylacyl or polyenylacyl, C₂₋₁₂ alkylacyl, substituted or unsubstituted 6- to 14-membered aryl, substituted or unsubstituted 3- to 14-membered heterocyclyl, substituted or unsubstituted 7- to 12-membered bridged-ring group.

4. The compound, a pharmaceutically acceptable salt, a stereoisomer, a tautomer or an isomer mixture, a hydrate, a solvate or a prodrug thereof according to claim 2 or 3,
wherein, X is -CH₂- or -C(O)-.

5. The compound, a pharmaceutically acceptable salt, a stereoisomer, a tautomer or an isomer mixture, a hydrate, a solvate or a prodrug thereof according to any one of claims 3 to 5,
wherein, R₁ is hydrogen, C₃₋₁₀ alkyl, C₄₋₁₂ dienyl, phenyl-substituted C₁₋₄ alkyl or indol-3-yl-(CH₂)ₙ-C(O)-, wherein n is an integer between 0 and 4;
for example, R₁ is hydrogen, C₆₋₁₀ alkyl, C₈₋₁₂ dienyl, phenyl-substituted C₁₋₃ alkyl or indol-3-yl-(CH₂)ₙ-C(O)-, wherein n is an integer between 0 and 4;
for example, R₁ is hydrogen, n-hexyl, n-heptyl, n-octyl, n-nonyl, n-decyl, 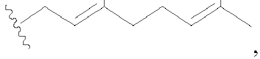 benzyl, phenylethyl, phenyl-n-propyl, phenyl-n-butyl, indol-3-yl-C(O)-, indol-3-yl-CH₂-C(O)-, indol-3-yl-(CH₂)₂-C(O)-, indol-3-yl-(CH₂)₃-C(O)- or indol-3-yl-(CH₂)₄-C(O)-;
for example, R₁ is hydrogen, 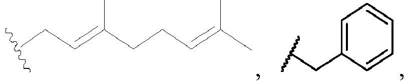
- CH₂(CH₂)₆CH₃ or

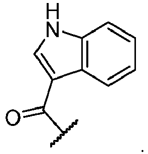

6. The compound, a pharmaceutically acceptable salt, a stereoisomer, a tautomer or an isomer mixture, a hydrate, a solvate or a prodrug thereof according to any one of claims 2 to 5, wherein,
R₃ is hydrogen, C₁₋₄ alkyl-substituted sulfonyl, C₁₋₄ alkyl-phenyl-substituted sulfonyl, C₄₋₁₂ dienylacyl, benzoyl substituted by one or more methoxy groups, phenyl-substituted C₁₋₄ alkyl, C₃₋₁₀ alkyl, C₂₋₁₂ alkylacyl, C₃₋₆ cycloalkyl, or indol-3-yl-(CH₂)ₙ-C(O)-, wherein n is an integer between 0 and 4,
for example, R₃ is hydrogen, C₁₋₃ alkyl-substituted sulfonyl, C₁₋₂ alkyl-phenyl-substituted sulfonyl, C₈₋₁₂ dienylacyl, benzoyl substituted by one or more methoxy groups, phenyl-substituted C₁₋₃ alkyl, C₆₋₁₀ alkyl, C₆₋₁₂ alkylacyl, C₃₋₄ cycloalkyl, or indol-3-yl-(CH₂)ₙ-C(O)-, wherein n is an integer between 0 and 4,
for example, R₃ is hydrogen, methylsulfonyl, ethylsulfonyl, propylsulfonyl, butylsulfonyl, p-toluenesulfonyl, p-ethylbenzenesulfonyl, p-n-propylbenzenesulfonyl, 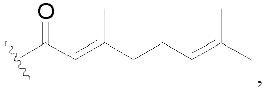 p-methoxybenzoyl, 3,4-dimethoxybenzoyl, 3,4,5-trimethoxybenzoyl, undecanoyl, decanoyl, nonanoyl, octanoyl, heptanoyl, hexanoyl, valeryl, butyryl, benzyl, phenylethyl, phenyl-n-propyl, phenyl-n-butyl, cyclopropyl, cyclobutyl, n-hexyl, n-heptyl, n-octyl, n-nonyl, n-decyl,
for example, R₃ is hydrogen, 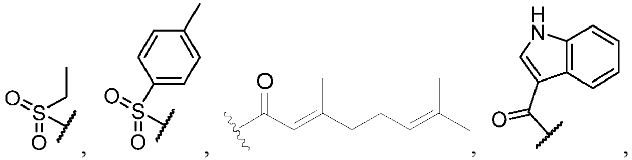 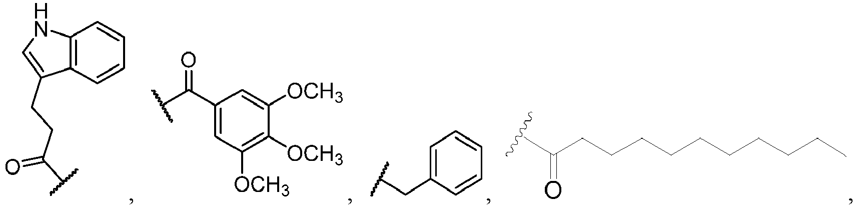 cyclopropyl,
- CH₂(CH₂)₆CH₃ or -CH₂(CH₂)₄CH₃.

7. The compound, a pharmaceutically acceptable salt, a stereoisomer, a tautomer or an isomer mixture, a hydrate, a solvate or a prodrug thereof according to any one of claims 2 to 6, wherein,
R₄ is hydrogen, C₄₋₁₂ dienyl, C₄₋₁₂ dienylacyl, C₁₋₄ alkyl-substituted sulfonyl, C₂₋₁₀ alkylacyl, C₃₋₁₀ alkyl, C₃₋₆ cycloalkyl, C₁₋₄ alkyl-phenyl-substituted sulfonyl,
for example, R₄ is hydrogen, C₈₋₁₂ dienyl, C₈₋₁₂ dienylacyl, C₁₋₃ alkyl-substituted sulfonyl, C₁₋₂ alkyl-phenyl-substituted sulfonyl, C₆₋₁₀ alkyl, C₃₋₄ cycloalkyl,
for example, R₄ is hydrogen, methylsulfonyl, ethylsulfonyl, propylsulfonyl, butylsulfonyl, p-toluenesulfonyl, p-ethylbenzenesulfonyl, p-n-propylbenzenesulfonyl, n-hexyl, n-heptyl, n-octyl, n-nonyl, n-decyl, cyclopropyl, cyclobutyl, 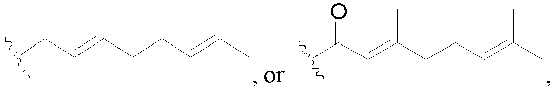
for example, R₄ is hydrogen, 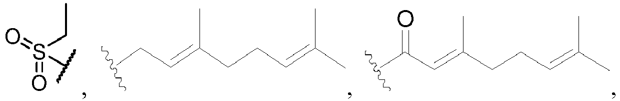 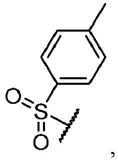 cyclopropyl, -CH₂(CH₂)₆CH₃ or -CH₂(CH₂)₄CH₃.

8. The compound, a pharmaceutically acceptable salt, a stereoisomer, a tautomer or an isomer mixture, a hydrate, a solvate or a prodrug thereof according to claim 1, wherein the compound is represented by Formula II, 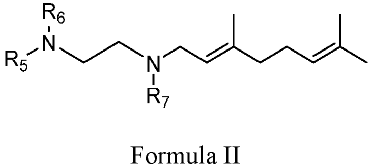 wherein,
R₅ is hydrogen, C₁₋₆ alkyl, C₃₋₁₄ cycloalkyl, 6- to 14-membered aryl, 3- to 14-membered heterocyclyl;
R₆ is hydrogen, C₁₋₆ alkyl, C₃₋₁₄ cycloalkyl, 6- to 14-membered aryl, 3- to 14-membered heterocyclyl, 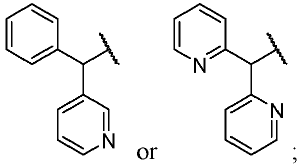
R₇ is hydrogen or substituted or unsubstituted sulfonyl.

9. The compound, a pharmaceutically acceptable salt, a stereoisomer, a tautomer or an isomer mixture, a hydrate, a solvate or a prodrug thereof according to any one of claims 2 to 7, wherein,
R₁ is hydrogen, or -CH₂(CH₂)₆CH₃, 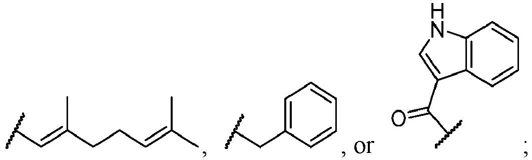
X is -CH₂- or -C(O)-;
R₄ is hydrogen, or 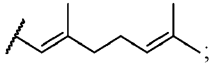
R₃ is hydrogen, -CH₂(CH₂)₄CH₃, -CH₂(CH₂)₆CH₃,

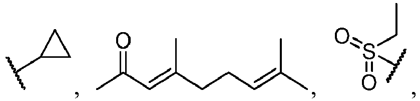

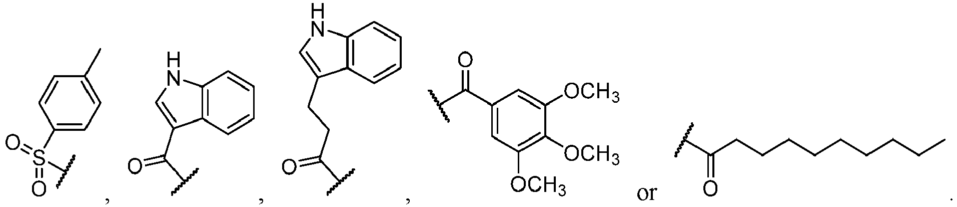

10. The compound, a pharmaceutically acceptable salt, a stereoisomer, a tautomer or an isomer mixture, a hydrate, a solvate or a prodrug thereof according to claim 8, wherein,
R₅ is hydrogen or C₁₋₄ alkyl;
R₆ is hydrogen, C₁₋₄ alkyl, 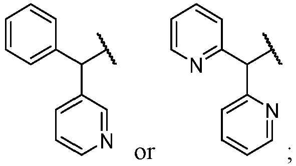
R₇ is hydrogen, C₁₋₄ alkyl-substituted sulfonyl or C₁₋₄ alkyl-phenyl-substituted sulfonyl, for example, R₅ is hydrogen, methyl, ethyl, n-propyl or isopropyl;
for example, R₆ is hydrogen, methyl, ethyl, n-propyl, isopropyl, 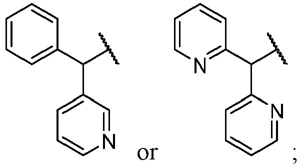
for example, R₇ is hydrogen, methylsulfonyl, ethylsulfonyl, propylsulfonyl, butylsulfonyl, p-toluenesulfonyl, p-ethylbenzenesulfonyl or p-n-propylbenzenesulfonyl.

11. The compound, a pharmaceutically acceptable salt, a stereoisomer, a tautomer or an isomer mixture, a hydrate, a solvate or a prodrug thereof according to claim 9, wherein,
R₅ is hydrogen;
R₆ is hydrogen, 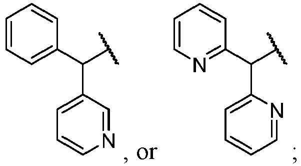
R₇ is hydrogen,

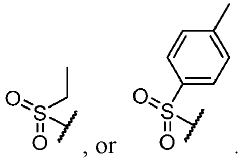

12. The compound, a pharmaceutically acceptable salt, a stereoisomer, a tautomer or an isomer mixture, a hydrate, a solvate or a prodrug thereof according to claim 1, wherein the compound is selected from the group consisting of:

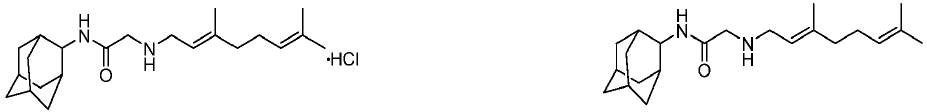

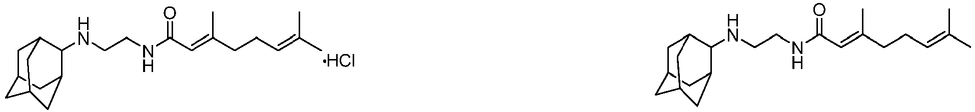

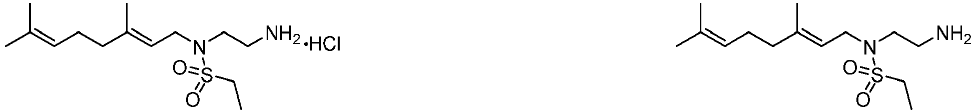

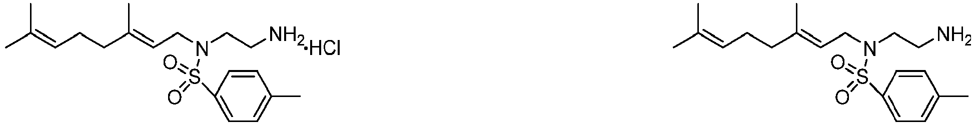

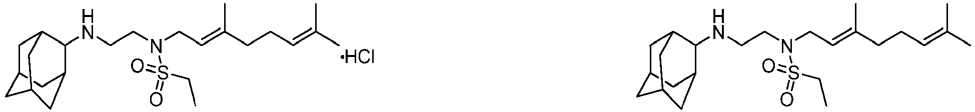

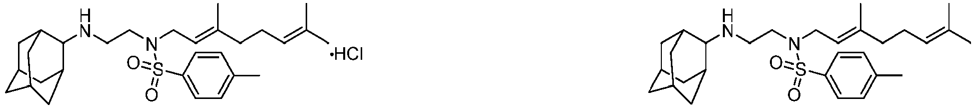

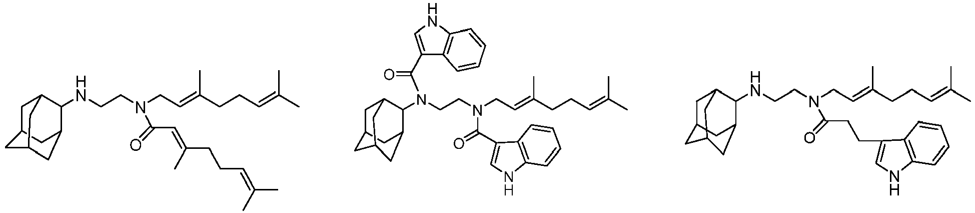

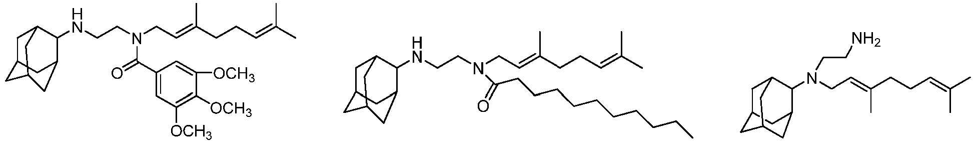

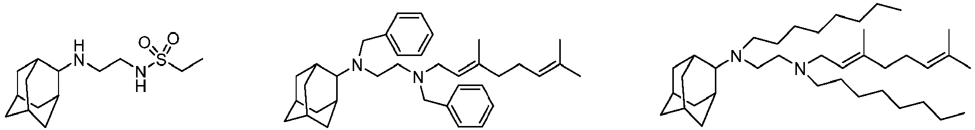

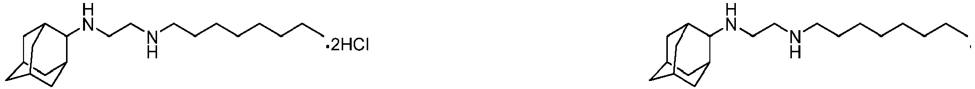

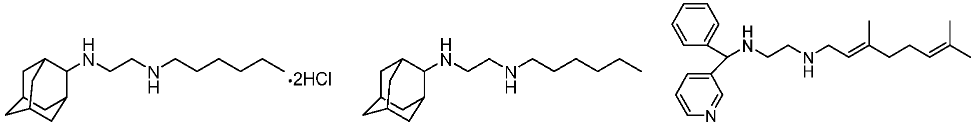

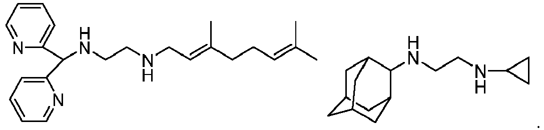

13. The compound, a pharmaceutically acceptable salt, a stereoisomer, a tautomer or an isomer mixture, a hydrate, a solvate or a prodrug thereof according to any one of claims 1 to 12, wherein the pharmaceutically acceptable salt is an inorganic acid salt such as hydrochloride, sulfate, phosphate, or organic acid salt such as methanesulfonate, trifluoromethanesulfonate, acetate, trifluoroacetate, benzoate, of the compound.

14. A pharmaceutical composition, comprising the compound, a pharmaceutically acceptable salt, a stereoisomer, a tautomer or an isomer mixture, a hydrate, a solvate or a prodrug thereof according to any one of claims 1 to 12, and optionally one or more pharmaceutically acceptable carriers or excipients.

15. The compound, a pharmaceutically acceptable salt, a stereoisomer, a tautomer or an isomer mixture, a hydrate, a solvate or a prodrug thereof according to any one of claims 1 to 12, for use in inhibiting the growth or reproduction of *Mycobacterium tuberculosis* (such as drug-resistant *Mycobacterium tuberculosis)* or for use in treating a tuberculosis.

16. Use of the compound, a pharmaceutically acceptable salt, a stereoisomer, a tautomer or an isomer mixture, a hydrate, a solvate or a prodrug thereof according to any one of claims 1 to 12 in manufacture of a medicament for inhibiting the growth or reproduction of *Mycobacterium tuberculosis* (such as drug-resistant *Mycobacterium tuberculosis)* or for treating a tuberculosis.

17. A method for treating tuberculosis, comprising administering to a subject in need a therapeutically effective amount of at least one of the compound, a pharmaceutically acceptable salt, a stereoisomer, a tautomer or an isomer mixture, a hydrate, a solvate or a prodrug thereof according to any one of claims 1 to 12.

18. A method for inhibiting the growth or reproduction of *Mycobacterium tuberculosis* (such as drug-resistant *Mycobacterium tuberculosis)* in vivo or in vitro, comprising contacting the *Mycobacterium tuberculosis* (such as drug-resistant *Mycobacterium tuberculosis)* with the compound, a pharmaceutically acceptable salt, a stereoisomer, a tautomer or an isomer mixture, a hydrate, a solvate or a prodrug thereof according to any one of claims 1 to 12.
